# FASCICULE DE BREVET EUROPEEN

(11) **EP 1 745 123 B1**
(45) Date de publication et mention de la délivrance du brevet: **11.07.2012**
(21) Numéro de dépôt: 05763706.8
(22) Date de dépôt: 02.05.2005
(51) Int. Cl.: C12N 1/18, A61K 51/04, C07B 59/00, C12P 33/00

(54) **SOUCHES DE LEVURE PRODUISANT DU CHOLESTEROL ET LEURS APPLICATIONS**
CHOLESTERINPRODUZIERENDE HEFESTÄMME UND DEREN VERWENDUNG
CHOLESTEROL-PRODUCING YEAST STRAINS AND USES THEREOF

(30) Priorité: 06.05.2004 FR 0404890
(43) Date de publication de la demande: 24.01.2007
(60) Demande divisionnaire: 10186024.5
(73) Titulaire: Aventis Pharma S.A., 92160 Antony (FR)
(72) Inventeur: POMPON, Denis, F-91190 Gif Sur Yvette (FR); DUMAS, Bruno, F-91300 Massy (FR); SPAGNOLI, Roberto, F-75016 Paris (FR)
(74) Mandataire: Bouvet, Philippe
(86) Numéro de dépôt international: PCT/FR2005/001090
(87) Numéro de publication internationale: WO 2005/121315

(56) Documents cités:
- EP-A- 0 727 489
- XU S ET AL: "BIOSYNTHESIS OF CHOLESTEROL IN THE YEAST MUTANT ERG6" BIOCHEMICAL AND BIOPHYSICAL RESEARCH COMMUNICATIONS, vol. 155, no. 1, 1988, pages 509-517, XP009039462 ISSN: 0006-291X
- MCCORKINDALE N J ET AL: "A COMPARISON OF THE TYPES OF STEROL FOUND IN SPECIES OF THE SAPROLEGNIALES AND LEPTOMITALES WITH THOSE FOUND IN SOME OTHER PHYCOMYCETES" PHYTOCHEMISTRY (OXFORD), vol. 8, no. 5, 1969, pages 861-867, XP002350564 ISSN: 0031-9422
- OSTLUND JR R E ET AL: "[<13>C]cholesterol as a tracer for studies of cholesterol metabolism in humans" JOURNAL OF LIPID RESEARCH 1993 UNITED STATES, vol. 34, no. 10, 1993, pages 1825-1831, XP002350566 ISSN: 0022-2275
- DATABASE WPI Section Ch, Week 200436 Derwent Publications Ltd., London, GB; Class B04, AN 2004-382697 XP002350568 & JP 2004 141125 A (MITSUBISHI CHEM CORP) 20 mai 2004 (2004-05-20)
- FABIAN F MOEBIUS ET AL: "Molecular cloning and expression of the human delta7-sterol reductase" PROCEEDINGS OF THE NATIONAL ACADEMY OF SCIENCES OF USA, NATIONAL ACADEMY OF SCIENCE, WASHINGTON, DC, US, vol. 95, no. 4, 17 février 1998 (1998-02-17), pages 1899-1902, XP002087882 ISSN: 0027-8424
- BAE SOO-HAN ET AL: "Cholesterol biosynthesis from lanosterol: Molecular cloning, tissue distribution, expression, chromosomal localization, and regulation of rat 7-dehydrocholesterol reductase, a Smith-Lemli-Opitz syndrome-related protein" JOURNAL OF BIOLOGICAL CHEMISTRY, vol. 274, no. 21, 21 mai 1999 (1999-05-21), pages 14624-14631, XP002350565 ISSN: 0021-9258
- DUPORT C ET AL: "Self-sufficient biosynthesis of pregnenolone and progesterone in engineered yeast" NATURE BIOTECHNOLOGY, NATURE PUBLISHING, US, vol. 16, no. 2, 1 février 1998 (1998-02-01), pages 186-189, XP002084824 ISSN: 1087-0156

### SEQUENCE LISTING

<110> Aventis pharma S.A.
<120> SOUCHES DE LEVURE PRODUISANT DU CHOLESTEROL ET LEURS APPLICATIONS
<130> PRJ02025
<160> 7
<170> PatentIn version 3.1
<210> 1
   <211> 63
   <212> DNA
   <213> Artificial
<400> 1
   cctagcgacg aaaagcatca ttggagtgaa taacttggac ttaccattct tagcattttg 60
   acg 63
<210> 2
   <211> 68
   <212> DNA
   <213> Artificial
<400> 2
   gcataagagt gaaacagaat tgagaaaaag acaggcccaa ttcaaattcg ggtcgaaaaa 60
   agaaaagg 68
<210> 3
   <211> 25
   <212> DNA
   <213> Artificial
<400> 3
   agggccgaac aaagccccga tcttc 25
<210> 4
   <211> 21
   <212> DNA
   <213> Artificial
<400> 4
   ggcaaaccga ggaactcttg 21
<210> 5
   <211> 36
   <212> DNA
   <213> Artificial
<400> 5
   tatatagcgg ccgctttcgc tgattaatta ccccag 36
<210> 6
   <211> 34
   <212> DNA
   <213> Artificial
<400> 6
   tatatagcgg ccgcgagaag tgacgcaagc atca 34
<210> 7
   <211> 270
   <212> DNA
   <213> Artificial
<400> 7
   tagcgtggat ggccaggcaa ctttagtgct gacacataca ggcatatata tatgtgtgcg     60
   acgacacatg atcatatggc atgcatgtgc tctgtatgta tataaaactc ttgttttctt    120
   cttttctcta aatattcttt ccttatacat taggtccttt gtagcataaa ttactatact    180
   tctatagaca cgcaaacaca aaggaattga caagtttgta caaaaaagca ggctaaaaaa    240
   tggaacctgc cgtgtcgctg gccgtgtgcg                                     270

## Description

La présente invention concerne la production de cholestérol chez des organismes du règne des *Fungi.*

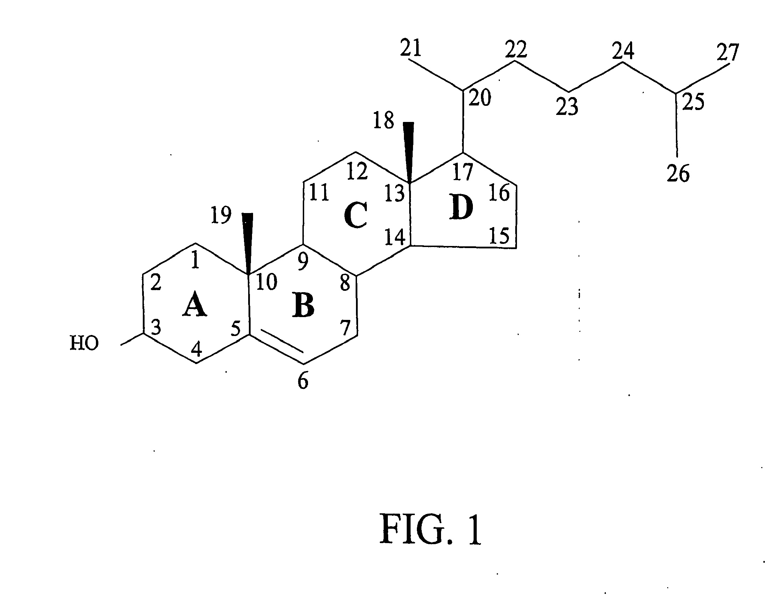

Le cholestérol (cf. figure 1) est le stérol animal le plus important. Il est un composant fondamental des membranes cellulaires, dont il contrôle la fluidité, et est présent dans tous les tissus animaux et particulièrement dans le tissu nerveux.

Le cholestérol est un produit d'intérêt industriel important. Ainsi, il est communément utilisé dans l'industrie cosmétique. Il est également utilisé dans l'industrie pharmaceutique par exemple dans l'administration de médicament (« Drug delivery ») ainsi que dans la culture cellulaire.

Le cholestérol est aussi utilisé dans la synthèse industrielle de vitamine D₃. Cette vitamine est ensuite utilisée pour supplémenter la nourriture humaine (dans les produits laitiers par exemple) et animal. Le cholestérol est également avantageusement utilisé comme additif dans la nourriture animal, notamment dans la nourriture destinée aux crevettes d'élevages.

Actuellement, l'immense majorité du cholestérol commercialisé est extrait à partir de tissu animal (une infime quantité est produite par synthèse chimique). Deux grandes sources de départ sont utilisées pour l'extraction du cholestérol : la moelle épinière de bovins et la lanoline qui est la graisse naturelle de la laine de mouton.

L'utilisation de tissu animal comme produit de départ n'est pas sans poser de problèmes. Ainsi, les récents problèmes liés à la transmission du prion responsable de la tremblante du mouton aux bovins (maladie appelée ESB (Encéphalopathie Spongiforme Bovine) chez les bovins) ont rappelé la nécessité de prudence lors de l'utilisation de tissu animal comme matière première. Cependant, malgré les mesures prises, le risque de transmission d'agent pathogène ne peut pas être totalement exclu. Il serait donc extrêmement avantageux de disposer d'une source de cholestérol ne provenant pas d'un tissu animal.

Xu, S. et al (1988) décrivent une souche de levure présentant une mutation du gène erg6 et dont l'enzyme 24-C-méthyltransférase est inactivée. Cette levure exprime les enzymes naturelles 7-déhydrocholestérol réductase et 3β-hydroxystérol Δ24-réductase. Elle produit des traces de cholestérol.

La présente invention a pour but de fournir une source de cholestérol abondante et sûre d'un point de vue sanitaire. Les inventeurs ont montré de manière surprenante qu'il est possible de détourner la production naturelle d'ergostérol chez les *Fungi* pour produire du cholestérol.

### Description générale de l'invention

L'invention concerne une souche de levure produisant de manière autonome du cholestérol à partir d'une source de carbone simple
caractérisée en ce qu'elle exprime les enzymes 7-déhydrocholestérol réductase et 3β-hydroxystérol Δ24-réductase et dans laquelle l'enzyme stérol 24-C méthyltransferase à été inactivée. Plus particulièrement, l'invention concerne un organisme tel que défini ci-dessus dans lequel l'enzyme C-22 stérol désaturase a en outre été inactivée.

Un autre aspect de l'invention concerne une souche de levure telle que définie ci-dessus caractérisée en ce que l'expression des enzymes 7-déhydrocholestérol réductase et 3β-hydroxystérol A24-réductase est obtenue par transformation de l'organisme.

L'invention concerne également un organisme du règne des *Fungi* tel que défini ci-dessus caractérisé en ce que l'inactivation de l'enzyme stérol 24-C-méthyltransferase est réalisée par inactivation génique et/ou l'inactivation de l'enzyme C-22 stérol désaturase est réalisée par inactivation génique.

Un autre aspect de l'invention concerne une souche de levure telle que définie ci-dessus caractérisée en ce qu'elle est choisie dans le phylum des *Ascomycete,* plus particulièrement dans le sous-phylum des *Saccharomycotina,* encore plus particulièrement dans la classe des *Saccharomycetes* ou des *Schizosaccharomycetes,* encore plus particulièrement dans l'ordre des *Saccharomycetales* ou des *Schizosaccharomycetales,* encore plus particulièrement dans la famille des *Saccharomycetaceae* ou des *Schizosaccharomycetaceae,* encore plus particulièrement dans le genre *Saccharomyces* ou *Schizosaccharomyces.*

Un autre aspect de l'invention concerne une levure de l'espèce *Saccharomyces cerevisiae* ou *Schizosaccharomyces pombe.*

L'invention concerne également un procédé de production de cholestérol d'origine non animal comprenant la culture d'un organisme tel que défini ci-dessus. Plus particulièrement, ce procédé est caractérisé en ce que l'étape de culture de l'organisme est suivie d'une étape d'extraction du cholestérol. De manière préférée, l'extraction du cholestérol est effectuée par un solvant non miscible dans l'eau.

Plus particulièrement, le procédé tel que défini ci-dessus est caractérisé en ce que l'on effectue une étape de saponification avant l'extraction du cholestérol. Encore plus particulièrement, le procédé tel que défini ci-dessus est caractérisé en ce que l'on effectue une étape de broyage mécanique des cellules avant la saponification ou l'extraction du cholestérol.

Un autre aspect de l'invention concerne l'utilisation d'une souche de levure telle que définie ci-dessus pour la production de cholestérol, ou de l'un de ses d'intermédiaires métaboliques, ou d'un mélange de stérols marqué au ¹³C ou au ¹⁴C.

L'invention est également relative à un procédé de production de cholestérol, ou de l'un de ses intermédiaires métaboliques, ou d'un mélange de stérols marqué au ¹³C ou au ¹⁴C comprenant les étapes suivantes :
- culture d'un organisme du règne des *Fungi* telle que défini ci-dessus sur un substrat marqué au ¹³C ou au ¹⁴C et,
- extraction dudit cholestérol, ou de l'un de ses intermédiaires métaboliques, ou du mélange de stérols.

L'invention concerne également un procédé de fabrication d'un mélange isotopique de cholestérol, d'intermédiaires ou de métabolites du cholestérol, marquées en différentes positions à l'aide de marqueurs isotopiques, comprenant la mise en culture d'un organisme du règne des *Fungi* telle que défini ci-dessus sur un substrat marqué puis sur un substrat non marqué, les durées de culture sur chacun de ces substrats étant choisies afin d'obtenir un profil isotopique défini.

### Description détaillée de l'invention

La présente invention concerne la production de cholestérol chez des organismes du règne des *Fungi.* Chez les *Fungi,* on ne trouve pas de cholestérol à l'état naturel, ce dernier étant un stérol animal. Le stérol majoritaire des membranes cellulaires de ces organismes est l'ergostérol.

La présente invention permet de réaliser la synthèse de cholestérol, par la multiplication de levures en présence d'une source de carbone simple. La méthode proposée par la présente invention permet donc d'obtenir une grande quantité de cholestérol, à faible coût, puisque le procédé met en oeuvre la culture d'organismes du règne des *Fungi* et l'ajout d'une source de carbone simple, facilement disponible dans le commerce.

Par source de carbone simple, selon la présente invention, on entend des sources de carbone utilisables par l'homme du métier pour la croissance normale d'une levure. On entend désigner notamment les différents sucres assimilables, tels le glucose, le galactose ou le saccharose, ou les mélasses, ou les sous-produits de ces sucres. Une source de carbone simple tout particulièrement préférée est l'éthanol et le glycérol.

Le fait que la production soit effectuée de manière autonome signifie qu'il n'est pas besoin de rajouter de substrats pour obtenir le cholestérol, mais que l'organisme peut le produire uniquement à partir de la source de carbone simple de départ. Il est aussi clair que la souche peut produire le cholestérol, par utilisation d'un substrat situé en amont dans la voie métabolique, dans la mesure où la souche de l'organisme selon la présente invention contient tous les gènes nécessaires à la complétion de la voie métabolique de production du cholestérol.

L'invention concerne notamment une levure génétiquement modifiée produisant de manière autonome du cholestérol à partir d'une source de carbone simple.

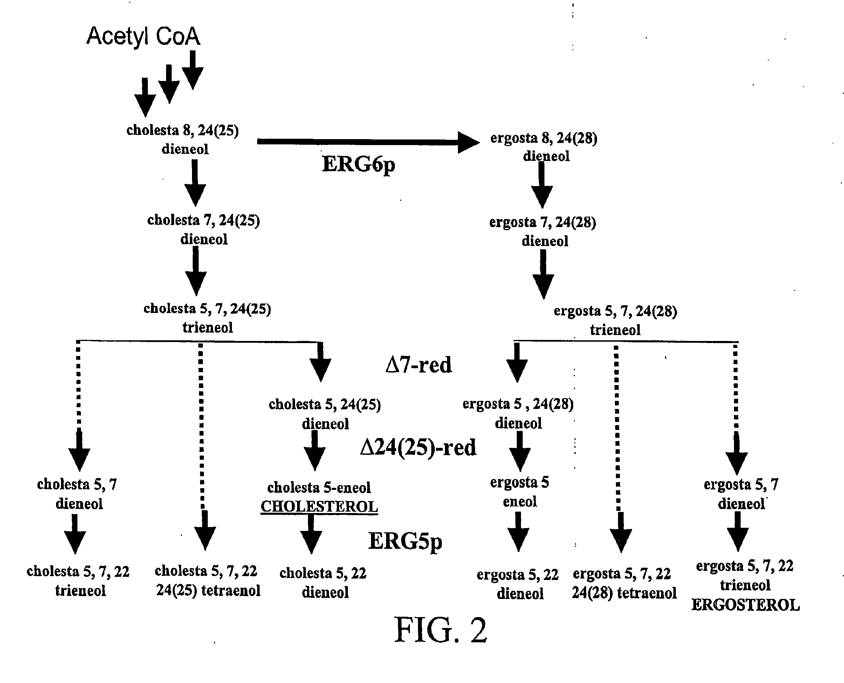

Un certain nombre de modifications génétiques de la levure peuvent être effectuées afin de détourner la voie métabolique naturelle de production de l'ergostérol vers la production de cholestérol. La présente invention concerne ainsi une levure génétiquement modifiée exprimant les enzymes 7-déhydrocholestérol réductase et 3β-hydroxystérol Δ24-réductase. La souche de levure ainsi modifiée produit du cholestérol. La demanderesse a en effet pu modéliser, grâce aux résultats obtenus (cf. la partie exemple de la présente demande), la voie métabolique conduisant à l'ergostérol et à certains de ses dérivés (cf. figure 2). L'expression des enzymes 7-déhydrocholestérol réductase et 3β-hydroxystérol A24-réductase chez le *fungus S. cerevisiae* peut permettre la production de cholestérol, en détournant une partie de la voie de biosynthèse de l'ergostérol.

L'enzyme 7-déhydrocholestérol réductase porte le numéro EC: 1.3.1.21 dans la classification internationale des enzymes (Enzyme Classification). Elle est également dénommée delta-5,7-stérol-delta-7-réductase, 7-DHC réductase ou Stérol delta-7-réductase et sera également dénommée Delta-7 stérol réductase, Delta-7Red, Delta 7 Réductase ou Δ7-réductase dans la suite de ce document. Cette enzyme catalyse à l'état naturel chez les plantes par exemple la réduction NADPH dépendante du delta 5, 7 cholestadienol en delta 5 cholestaenol ou la réduction d'intermédiaires stéroliques possédant la double liaison en 7-8 (Taton and Rahier, 1991). Le gène codant pour l'enzyme 7-déhydrocholestérol réductase a été isolé pour la première fois chez la plante *Arabidopsis thaliana,* l'isolement du gène correspondant et l'expression de cette enzyme chez la levure *Saccharomyces cerevisiae* est décrite dans le brevet EP 727 489. Les séquences de ce gène et de la protéine sont accessibles au numéro d'accession GenBank suivant : U49398 (Lecain et al., 1996).

Un certain nombre d'homologues de ce gène ont été décrits chez d'autres espèces. Il s'agit par exemple du gène homologue chez l'homme (dont la séquence nucléotidique est accessible au numéro GenBank AF034544, la séquence protéique au numéro GenBank : AAC05086) (Moebius et al., 1998). Du gène homologue chez le rat *Rattus norvegicus* (dont la séquence nucléotidique est accessible au numéro GenBank : AB016800, la séquence protéique au numéro GenBank : BAA34306). Des gènes homologues ont été également identifiés chez la poule *Gallus gallus* avec la référence Genbank BM490402 ou chez le crapaud *Xenopus laevis* avec la référence Genbank BI315007 ou le poisson zèbre *Danio rerio* avec la référence Genbank BQ132664. On trouve également un gène codant pour une activité delta7 stérol réductase chez les plantes comme le riz *Oryza sativa* avec la référence Genbank CA753545, la pomme de terre *Solanum tuberosum* avec la préférence Genbank BF342071. Ce gène codant pour une activité delta7 stérol réductase peut être également trouvé chez le protiste *Mastigomoeba balamuthi* avec la référence Genbank BE636562.

L'homme du métier pourra isoler aisément d'autres gènes homologues codant pour l'enzyme 7-déhydrocholéstérol réductase dans d'autres organismes. Il pourra notamment se référer à la méthode de clonage décrite à l'exemple 1 du brevet EP 727 489, qui décrit une méthode de clonage permettant d'isoler un ADNc codant pour une protéine ayant une activité delta-5,7-stérol-delta-7-réductase. L'homme du métier pourra également aisément déterminer l'activité 7-déhydrocholestérol réductase des protéines correspondantes notamment en utilisant le test d'activité également décrit à l'exemple 1 du brevet EP 727 489.

L'expression de l'enzyme 7-déhydrocholestérol réductase chez un organisme du règne des *Fungi* selon l'invention peut être obtenue par tout moyen connu de l'homme du métier. Il peut s'agir en particulier de la transformation de l'organisme par une construction comportant une cassette d'expression constituée d'un promoteur de transcription de préférence homologue, de la phase ouverte de lecture codant pour l'enzyme 7-déhydrocholestérol réductase et d'un terminateur de transcription adapté selon les règles habituelles connues de l'homme du métier. Comme promoteur homologue, on utilisera en général un promoteur adéquat pour permettre une expression suffisante et fonctionnelle de la protéine hétérologue. Le promoteur peut être par exemple le promoteur PGK, le promoteur ADH, le promoteur CYC1, le promoteur GAL10/CYC1, le promoteur TDH3 ou le promoteur TPI. Le terminateur peut être par exemple le terminateur du gène de la phosphoglycérate kinase (PGK). La dite cassette d'expression peut être intégrée sous forme d'une ou de plusieurs copies dans le génome nucléaire ou mitochondrial de l'hôte, ou porté par une structure artificielle du type chromosome artificiel de levure (YAC) ou être portée par un élément génétique épisomale comme un plasmide. Pour réaliser ce type d'expression, des levures du type *Yarrowia lipolitica, Kluyveromyces lactis* ou *Pichia pastoris* peuvent par exemple être utilisées.

Préférentiellement, l'enzyme 7-déhydrocholestérol réductase exprimée est l'enzyme de la plante *Arabidopsis thaliana* (un exemple de mode d'expression de cette enzyme chez la levure *Saccharomyces cerevisiae* est décrit dans le brevet EP 727 489). Il peut cependant s'agir de toute enzyme homologue ou non, naturelle ou artificielle présentant la même activité enzymatique.

L'enzyme 3β-hydroxystérol Δ24-réductase, également dénommée DHCR24 ou 24-déhydrocholesterol réductase, catalyse à l'état naturel la réduction du desmostérol (cholesta 5, 24 dienol ) ou des dérivés du lanostérol possédant une double liaison en 24-25 sur la chaîne latérale (par exemple le 14 desméthyl-lanostérol, le zymostérol ou le cholesta 7, 24 dienol), réduction nécessaire à la biosynthèse de cholestérol chez l'homme notamment (Waterham HR*. et al.,* 2001). Cette enzyme sera également dénommée delta 24-(25) stérol réductase, delta 24 stérol Réductase ou Δ24-réductase dans la suite de ce document.

Le gène codant pour l'enzyme 3β-hydroxystérol Δ24-réductase a été isolé pour la première fois chez l'homme, l'isolement du gène correspondant et l'expression de cette enzyme chez la levure *Saccharomyces cerevisiae* est décrite dans la publication Waterham HR. *et al.,* 2001. Les séquences de ce gène et de la protéine sont accessibles aux numéros d'accession GenBank suivant : NM_014762 et NP_055577.

Un certain nombre d'homologues de ce gène ont été décrits chez d'autres espèces. Il s'agit par exemple du gène homologue chez la souris (*Mus musculus*) (dont la séquence nucléotidique est accessible au numéro GenBank : NM_053272, la séquence protéique au numéro GenBank : NP_444502). Des homologues ont été décrits chez le ver *Caenorhabditis elegans* et notamment un ADN complémentaire avec la référence Genbank AF026214. Des séquences homologues ont également été décrites chez les plantes comme le coton *Gossypium hirsutum* avec la référence Genbank AAM 47602.1, le riz *Orysa sativa* avec la référence Genbank AAP53615, le pois *Pisum satinum* avec la référence Genbank AAK15493.

L'homme du métier pourra isoler aisément d'autres gènes homologues codant pour l'enzyme 3β-hydroxystérol Δ24-réductase dans d'autres organismes. Il pourra notamment se référer à la méthode de clonage décrite dans la publication Waterham HR. *et al.,* 2001. L'homme du métier pourra également aisément déterminer l'activité 3β-hydroxystérol Δ24-réductase des protéines correspondantes notamment en utilisant le test d'activité également décrit dans la publication (Waterham et al., 2001).

L'expression de l'enzyme 3β-hydroxystérol Δ24-réductase chez une levure selon l'invention peut être obtenue par tout moyen connu de l'homme du métier. Il peut s'agir en particulier des moyens décrits ci-dessus en ce qui concerne l'expression de l'enzyme 7-déhydrocholestérol réductase.

Préférentiellement, l'enzyme 3β-hydroxystérol Δ24-réductase exprimée est l'enzyme humaine. Un exemple d'isolement du gène correspondant et d'expression de cette enzyme chez la levure *Saccharomyces cerevisiae* est décrit dans la publication Waterham HR. *et al.,* 2001. Il peut cependant s'agir de toute enzyme homologue ou non, naturelle ou artificielle présentant la même activité enzymatique.

Avantageusement, les levures selon la présente invention expriment les enzymes 7-déhydrocholestérol réductase et 3β-hydroxystérol Δ24-réductase et présentent en outre une inactivation de l'enzyme stérol 24-C-méthyltransferase.

L'enzyme stérol 24-C-méthyltransferase porte le numéro EC-2.1.1.41 dans la classification internationale des enzymes (Enzyme Classification). Elle est également dénommée ERG6p, Delta(24)-methyltransferase, Delta(24)-stérol methyltransferase, Zymostérol-24-methyltransferase, S-adenosyl-4-methionine:stérol delta(24)-methyltransferase; SMT1, 24-stérol C-methyltransferase, S-adenosyl-L-methionine:delta(24(23))-stérol methyltransferase ou Phytostéroi methyltransferase. Cette enzyme catalyse à l' état naturel la méthylation en C-24 du zymostérol, conduisant à la formation de fécostérol.

Le gène codant pour l'enzyme stérol 24-C-méthyltransferase a été dénommé *Erg6* chez la levure *Saccharomyces cerevisiae.* La séquence de ce gène est accessible au numéro d'accession GenBank suivant : NC_001145. La séquence de la protéine correspondante est accessible au numéro d'accession GenBank suivant : NP_013706 (Bowman et al., 1997), (Goffeau et al., 1996).

Un certain nombre d'homologues de ce gène ont été décrits chez d'autres *Fungi.* Il s'agit par exemple du gène homologue chez *Schizosaccharomyces pombe* (dont la séquence nucléotidique est accessible au numéro GenBank Z99759, la séquence protéique au numéro GenBank : CAB16897) (Wood et al., 2002). Du gène homologue chez *Neurospora crassa* (dont la séquence nucléotidique est accessible au numéro GenBank : NCB24P7, la séquence protéique au numéro GenBank : CAB97289). Du gène homologue chez *Candida albicans* (dont la séquence nucléotidique est accessible au numéro Genbank: AF031941, la séquence protéique au numéro GenBank : AAC26626) (Jensen-Pergakes et al., 1998). Des gènes codant pour une enzyme homologue à ERG6 ont également été décrits chez *Candida lusitaniae* avec la référence Genbank AA021936.1 et également chez *Pneumocystis carinii* (Kaneshiro et al., 2002) ou chez *Kluveromyces lactis* (Ozier-Kalogeropoulos et al., 1998).

L'homme du métier pourra isoler aisément d'autres gènes homologues du gène *Erg6* dans les organismes du règne *Fungi.* L'homme du métier pourra également aisément déterminer l'activité stérol 24-C-méthyltransferase des protéines correspondantes notamment en utilisant comme test d'activité la complémention fonctionnelle d'une souche de levure disruptée pour ces gènes. La complémentation est alors attestée par la formation de stérols branchés en position 24 en particulier de stérols de type ergosta- portant un groupement méthylene en position 24-28. La présence d'une activité biologique stérol 24-C-méthyltransférase de type *ERG6* sera également déterminée *in vitro* grâce aux techniques développées par (McCammon et al., 1984) ou par Taylor et Parks (Taylor and Parks, 1978). D'autre part les stérols produits et substrat de l'enzyme ERG6 seront séparés par chromatographie en phase gazeuse selon la technique développée par Nes in (Methods in Enzymology Steroids and Isoprenoids Volume 111 part B, 1985 «A comparison of Methods for the Identification of Sterols » pp3-37).

La souche de levure selon la présente invention exprimant les enzymes 7-déhydrocholestérol réductase et 3β-hydroxystérol Δ24-réductase et présentant en outre une inactivation de l'enzyme stérol 24-C-méthyltransferase produit du cholestérol. La demanderesse a en effet pu déterminer que, de manière surprenante, l'inactivation de l'enzyme stérol 24-C-méthyltransferase bloques la voie de biosynthèse de l'ergostérol en amont et permet une production accrue en cholestérol par la souche de levure (cf. la partie exemple de la présente demande).

L'expression des enzymes 7-déhydrocholestérol réductase et 3β-hydroxystérol Δ24-réductase est réalisée comme décrit ci-dessus.

L'inactivation de l'enzyme stérol 24-C-méthyltransferase peut être réalisée par tout moyen connu de l'homme du métier. Il peut s'agir en particulier de l'introduction par mutagenèse d'une mutation non-sens, d'une insertion ou d'une délétion provoquant un changement de cadre de lecture dans le gène codant ladite protéine.

Il peut s'agir également de l'expression d'un ARN anti-sens complémentaire de l'ARN messager codant ladite protéine ou du système d'extinction génique connu de l'homme de l'art sous le nom de RNAi (small interfering RNA) et des systèmes enzymatiques associés si ceux-là n'existent pas naturellement chez l'hôte. La mutagénèse peut être effectuée dans la séquence codante ou dans une séquence non codante de manière à rendre inactive la protéine codée ou à en empêcher son expression ou sa traduction. La mutagenèse peut être effectuée *in vitro* ou *in situ*, par suppression, substitution, délétion et/ou addition d'une ou plusieurs bases dans le gène considéré ou par inactivation génique.

Il peut s'agir en particulier de l'introduction d'un ADN exogène dans la séquence codante ou promotrice (par exemple une cassette d'expression avec promoteur et/ou terminateur homologue et une partie codante hétérologue). La cassette d'expression permet avantageusement l'expression d'un marqueur de sélection. Il est également possible de modifier le promoteur du gène afin de réduire le niveau d'expression. Pour les *fungi*, l'inactivation se fait également par interruption de la séquence codante par la séquence codante d'un gène marqueur hétérologue ou homologue. Les principales techniques pour interrompre un gène de *fungi* sont décrites dans l'article de Johnston et al (2002) (Methods in Enzymology Volume 350 Edited by Christine Guthrie and Gerry Fink ; « Gène Disruption » ; M. Johnston, L. Riles, J. Hegemann pp290-315).

Avantageusement, les levures selon la présente invention expriment les enzymes 7-déhydrocholestérol réductase et 3β-hydroxystérol Δ24-réductase et présentent en outre une inactivation de l'enzyme C-22 stérol désaturase.

L'enzyme C-22 stérol désaturase est également dénommée ERG5p, Cyp61, cytochrome p-45061, stérol delta22-désaturase. Cette enzyme catalyse à l'état naturel la conversion de l'ergosta-5,7,24(28)-trienol en ergosta-5,7,22,24(28)-tétraenol en ajoutant une double liaison en position C22 (cf. figure 2).

Le gène codant pour l'enzyme l'enzyme C-22 stérol désaturase a été dénommé *Erg5* chez la levure *Saccharomyces cerevisiae.* La séquence de ce gène est accessible au numéro d'accession GenBank suivant : U34636. La séquence de la protéine correspondante est accessible aux numéros d'accession GenBank suivants : AAB06217 (Skaggs et al., 1996) ou P54781 (Bowman et al., 1997).

Un certain nombre d'homologues de ce gène ont été décrits chez d'autres *Fungi.* Il s'agit par exemple du gène homologue chez *Schizosaccharomyces pombe* (dont la séquence nucléotidique est accessible au numéro GenBank Z98974, la séquence protéique au numéro GenBank : CAB11640) (Wood et al., 2002). Du gène homologue chez *Symbiotaphrina buchneri* (dont la séquence nucléotidique est accessible au numéro GenBank: AB086896, la séquence protéique au numéro Genbank : BAC01142) (Noda and Koizumi, 2003). Du gène homologue chez *Symbiotaphrina kochii* (dont la séquence nucléotidique est accessible au numéro GenBank: AB086890, la séquence protéique au numéro GenBank : BAC01139) (Noda and Koizumi, 2003). Du gène homologue chez *Candida albicans* (dont la séquence nucléotidique est accessible au numéro GenBank : AL033396, la séquence protéique au numéro GenBank : CAA21953) (Tait et al., 1997). Le gène ERG5 a également été décrit chez *Candida lusitaniae* avec la référence Genbank AAO48601.

L'homme du métier pourra isoler aisément d'autres gènes homologues du gène *Erg5* dans les organismes du règne *Fungi.* L'homme du métier pourra également aisément déterminer l'activité C-22 stérol desaturase des protéines correspondantes notamment en utilisant le test d'activité décrit par Skaggs,B.A., *et al.,* 1996. Cette activité pourra également être mise en évidence par complémentation fonctionnelle d'une levure *S*. *cerevisiae* préalablement disruptée au niveau du gène erg5. Cette complémentation sera attestée par la présence dans la souche complémentée d'ergosta 5,7,22 trienol. L'activité C-22 stérol désaturase peut être mesurée *in vitro* en utilisant la méthode décrite par Kelly and Baldwin et al JBC (1997) après lyse des levures (Kelly et al., 1997).

La souche de levure selon la présente invention exprimant les enzymes 7-déhydrocholestérol réductase et 3β-hydroxystérol Δ24-réductase et présentant en outre une inactivation de l'enzyme C-22 stérol désaturase produit du cholestérol. La demanderesse a en effet pu déterminer que l'inactivation de l'enzyme C-22 stérol désaturase bloque avantageusement la conversion du cholestérol en cholesta 5, 22 diéneol et permet une stabilisation de la production en cholestérol (cf. la partie exemple de la présente demande). Ce blocage intervient aussi au niveau de la conversion du cholesta 5,7 dienol, un précurseur du cholesterol en cholesta 5,7,22 trienol, un précurseur du cholesta 5,22 dienol. De manière surprenante, l'enzyme C-22 stérol désaturase accepte en effet comme substrat le cholestérol qu'elle convertit en cholesta 5, 22 diéneol. Cette réaction parasite peut être éliminée par l'inactivation de l'enzyme -22 stérol désaturase, telle qu'il a pu être déterminé par la demanderesse.

L'expression des enzymes 7-déhydrocholestérol réductase et 3β-hydroxystérol Δ24-réductase est réalisée comme décrit ci-dessus. L'inactivation de l'enzyme C-22 stérol désaturase peut être réalisée par tout moyen connu de l'homme du métier. Il peut s'agir en particulier des méthodes décrites ci-dessus concernant l'inactivation de l'enzyme stérol 24-C-méthyltransferase.

Avantageusement, les levures selon la présente invention expriment les enzymes 7-déhydrocholestérol réductase et 3β-hydroxystérol Δ24-réductase et présentent en outre une inactivation de l'enzyme C-22 stérol désaturase et une inactivation de l'enzyme stérol 24-C-méthyltransferase. Ces souches présentent en effet les avantages cumulés des deux inactivations et sont des souches productrices de cholestérol.

L'expression des enzymes 7-déhydrocholestérol réductase et 3β-hydroxystérol Δ24-réductase et l'inactivation des enzymes C-22 stérol désaturase et stérol 24-C-méthyltransferase sont réalisées comme décrit ci-dessus.

Dans un mode de réalisation, le cholestérol est présent dans la souche d'organisme selon la présente invention dans une proportion supérieure à 20 %, de préférence 35 %, de façon la plus préférée 50 % ou plus du total des stérols produits par la souche selon l'invention (notamment les intermédiaires de synthèse).

Préférentiellement, les levures selon la présente invention sont choisis dans le phylum des *Ascomycetes,* de manière plus préférée, ils sont choisis dans le sous-phylum des *Saccharomycotina,* de manière encore plus préférée, ils sont choisis dans la classe des *Saccharomycetes* ou des *Schizosaccharomycetes,* de manière encore plus préférée ils sont choisis dans l'ordre des *Saccharomycetales* ou des *Schizosaccharomycetales.,* de manière encore plus préférée ils sont choisis dans la famille des *Saccharomycetaceae* ou des *Schizosaccharomycétaceae,* de manière encore plus préférée ils sont choisis dans le genre *Saccharomyces* ou *Schizosaccharomyces,* de manière tout à fait préférée les organismes du règne des *Fungi* selon l'invention appartiennent à l'espèce *Saccharomyces cerevisiae* ou *Schizosaccharomyces pombe.*

La présente invention concerne également un procédé de production de cholestérol d'origine non animal comprenant les étapes suivantes :
- on cultive une levure tel que décrit ci-dessus,
- on extrait le cholestérol produit par cet organisme.

L'extraction est basée sur le traitement de la levure par un solvant du cholestérol de préférence non miscible dans l'eau. Ce traitement peut être préférentiellement associé à une méthode quelconque de broyage mécanique des cellules. Plus préférentiellement la levure sera traitée avant extraction par le solvant, par un mélange de saponification destiné à libérer le cholestérol éventuellement lié à d'autres composants cellulaires comme en particulier les acides gras. Ce mélange de saponification pourra être constitué d'une base par exemple ammoniaque, soude, potasse dissoute dans de l'eau ou plus préférentiellement dans un solvant organique miscible dans l'eau comme par exemple le méthanol ou l'éthanol, ou un mélange solvant-eau. La saponification pourra être effectuée sans ou préférentiellement avec chauffage à une température de 60-120 °C, à pression atmosphérique ou sous-pression. L'extraction par le solvant non miscible dans l'eau pourra être remplacée par une extraction en phase solide sur une résine hydrophobe. Une méthode d'extraction des stérols est décrite par L. Parks et collaborateurs (1985) (Methods in Enzymology 111 Edited by L Rilling, L. Parks, C. Bottema, R. Rodriguez and Thomas Lewis pp333-339).

Le cholestérol brut ainsi obtenu pourra être purifié par toutes méthodes connues de l'homme de l'art, en particulier celle décrite par Boselli E, Velazco V, Caboni Mf et Lercker G J Chromatogr A. 2001 May 11;917(1-2):239-44.

D'autres méthodes pourront également être utilisées comme celle décrite pour l'extraction du cholestérol à partir de laine de mouton. L'homme du métier pourra notamment se référer aux méthodes décrites dans les brevets américains US 2,688,623 ou US 2,650,929 ou dans les brevets britanniques GB690879, GB646227 ou GB613778.

Un autre aspect de l'invention concerne l'utilisation des souches selon la présente invention afin d'obtenir le cholestérol ou l'un de ses d'intermédiaires métaboliques, ou un mélange de stérols marqué. On entend par intermédiaire métabolique du cholestérol, notamment les stérols spécifiés dans la figure 2. Il peut s'agir notamment de cholesta 8, 24(25) dieneol, cholesta 7, 24(25) dieneol, cholesta 5, 7, 24(25) trieneol, cholesta 5, 24(25) dieneol, cholesta 5, 22 dieneol.

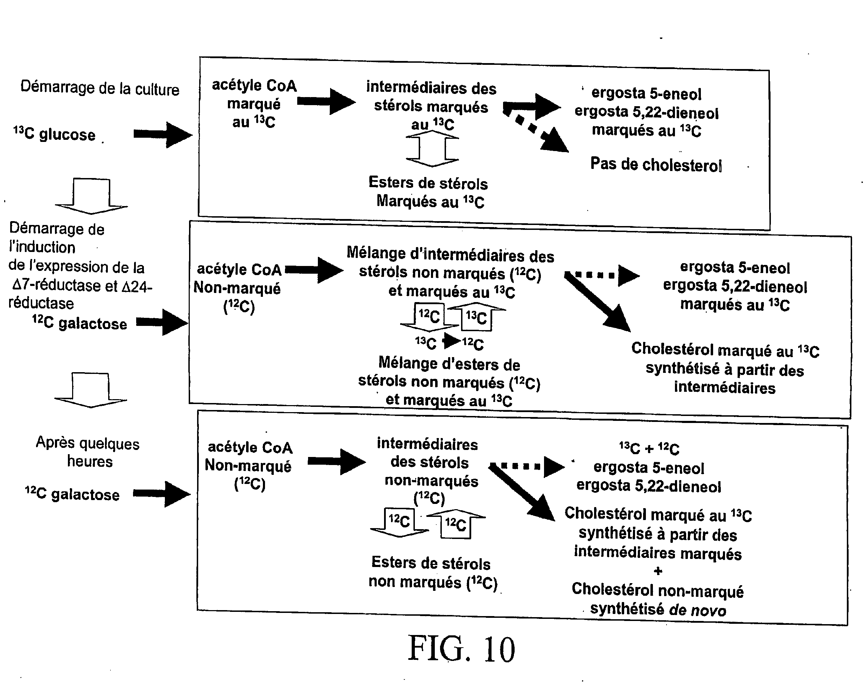

Le principe de l'obtention d'un cholestérol marqué est décrit à la figure 10. Cette manipulation consiste à tout d'abord faire croître la levure sur un substrat totalement marqué. Les cellules sont ensuite mises en culture sur un substrat non marqué. Il y a ainsi changement de marquage isotopique de la source de carbone, il s'ensuit la synthèse *de novo* d'intermédiaires métaboliques puis de stérol, incluant le cholestérol et comportant un changement progressif de marquage. Il s'agit donc d'un profil complexe, mais parfaitement déterminable expérimentalement, qui représente une signature isotopique unique qui dépend à la fois :
- 1) du protocole de marquage et en particulier des durées et des conditions de culture en substrat marqué et non marqué.
- 2) de la structure génétique précise de la souche utilisée
- 3) du temps précis d'arrêt des cultures

Une fois la culture arrêtée ( par exemple par lyse cellulaire ou par arrêt de la culture en présence de concentration sub-létale de produits antifongiques cytotoxiques ou cytostatiques ), le cholestérol marqué ou l'un de ses d'intermédiaires métaboliques, ou un mélange de stérols marqué est extrait et purifié comme décrit ci-dessus.

Le profil isotopique du cholestérol marqué ou de l'un de ses intermédiaires métaboliques, ou du mélange de stérols marqué a plusieurs propriétés uniques :
- 1) il est modulable à volonté en jouant sur les conditions de culture, la souche utilisée et le stérol choisi. Un registre de marqueurs unique peut donc être produit.
- 2) il est « combinable » à savoir que plusieurs signatures isotopiques correspondants à plusieurs stérols uniques marqués par des profils isotopiques eux-même modulables peuvent être combinés pour former un « alphabet moléculaire »
- 3) il est reproductible et facile à déterminer expérimentalement.
- 4) il correspond à un mélange moléculaire traceur facile à isoler, stable, incolore et inodore, non volatile, non toxique et incorporable à des aliments, médicament, additifs ou autres produits assimilables par l'homme.
- 5) il est infalsifiable sauf à disposer des souches recombinantes spécifiques et des conditions très précises de marquage, de culture et d'extraction. De plus la connaissance de la signature isotopique ne permet pas de remonter aux paramètres qui ont permis de la produire.

Ainsi, un « alphabet isotopique » infalsifiable, d'usage général et incorporable dans tout type de produits, y compris consommables peut être obtenu facilement grâce à la présente invention. Les « mots isotopiques » constituables à partir d'un tel alphabet sont en nombre quasi illimité en mettant à profit à la fois les profils de marquage et les différents types de stérols. L'incorporation de telles signatures au sein de produits les plus variés constitue donc une méthode unique de marquage infalsifiable, contrairement par exemple aux signatures ADN qui peuvent être reproduites une fois connues. La signature peut être lue par ailleurs de manière non destructive, par exemple par ionisation laser suivie d'analyse par spectrométrie de masse (MALDI-TOF ou assimilé).

L'utilisation de substrat marqué au ¹³C en lieu et place des sources de carbone non marquée pour la culture de levures selon l'invention permet la synthèse de stérols et en particulier de cholestérol très fortement marqué (comprenant au moins 95 % de carbone ¹³C). La préparation de stérols et de cholesterol radioactif ¹⁴C est également possible par la même approche. Le procédé peut aussi être incorporé à des souches de levures produisant des stéroïdes et notamment de l'hydrocortisone (cf. demande de brevet WO 02/061109) pour produire des stéroïdes marqués au ¹³C ou au ¹⁴C par exemple pour des tests RIA.

### Légende des figures

Figure 1 : Formule chimique du cholestérol, ainsi que la nomenclature généralement utilisée pour la numérotation des différents carbones et le nom des différents cycles. Les quatre cycles de la molécule de cholestérol sont dénommés respectivement A, B, C et D et les carbones sont numérotés de 1 à 27.
Figure 2 : Schéma simplifié de la partie tardive de la voie de biosynthèse des stérols de types ergosta- et cholesta- dans les levures naturelles ou modifiées. Le schéma n'est pas exhaustif mais permet de définir les étapes faisant intervenir les enzymes citées dans ce document. Les protéines ERG2p, ERG3p, ERG5p et ERG6p sont des protéines de *fungus* ou de levure, tandis que les protéines Delta-7Red (Delta-7 stérol réductase), Delta 24-(25)Red (Delta 24-(25) stérol réductase) sont des protéines hétérologues d'origine de mammifères ou de plantes.

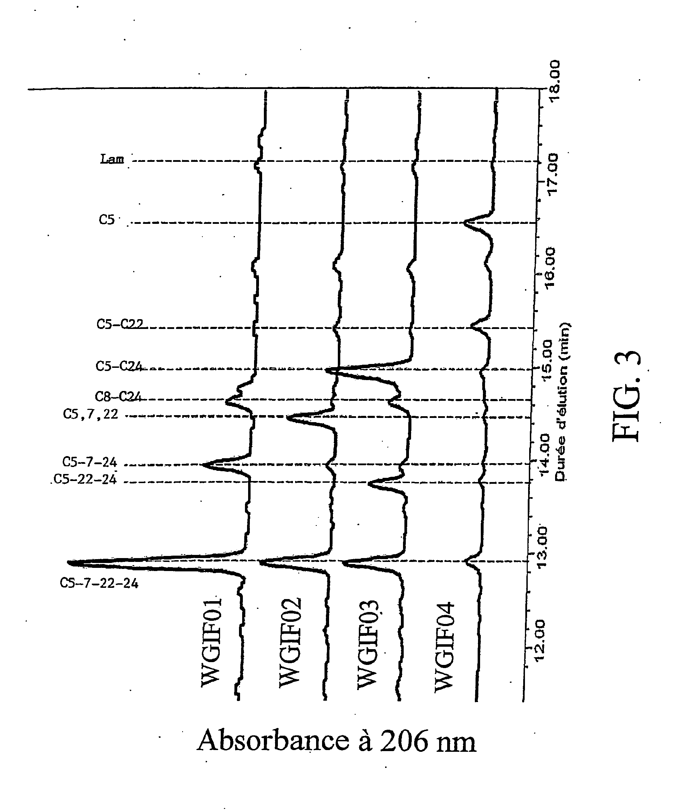

Figure 3. Profil comparé en HPLC avec détection UV à 206 nm des stérols libres des souches dérivées de la souche BMA64 et identification de ces stérols. Les souches étudiées sont les suivantes : WGIF01 (souche BMA64 disruptée dans le gène *erg6* (cf. exemple 1)), WGIF02 (souche BMA64 disruptée dans le gène *erg6* et exprimant la Δ24-réductase, Exemple 12), WGIF03 (souche BMA64 disruptée dans le gène *erg6* et exprimant la Δ7-réductase, Exemple 13), WGIF04 (souche BMA64 disruptée dans le gène *erg6* et exprimant la Δ7-réductase et la Δ24-réductase, Exemple14). C5 : cholesta 5 eneol (cholestérol) ; C5,22: cholesta 5,22 diéneol ; C5, 24: cholesta 5,24 diéneol (desmostérol) ; C8,24: cholesta 8,24 diéneol (zymostérol); C5,7,22: cholesta 5,7,22 triénol; C5.7, 24: cholesta 5,7,24 triénol; C5,22,24: cholesta 5,22,24 triénol; C5,7,22,24: cholesta 5,7,22,24 tetraénol ; lan : lanostérol.

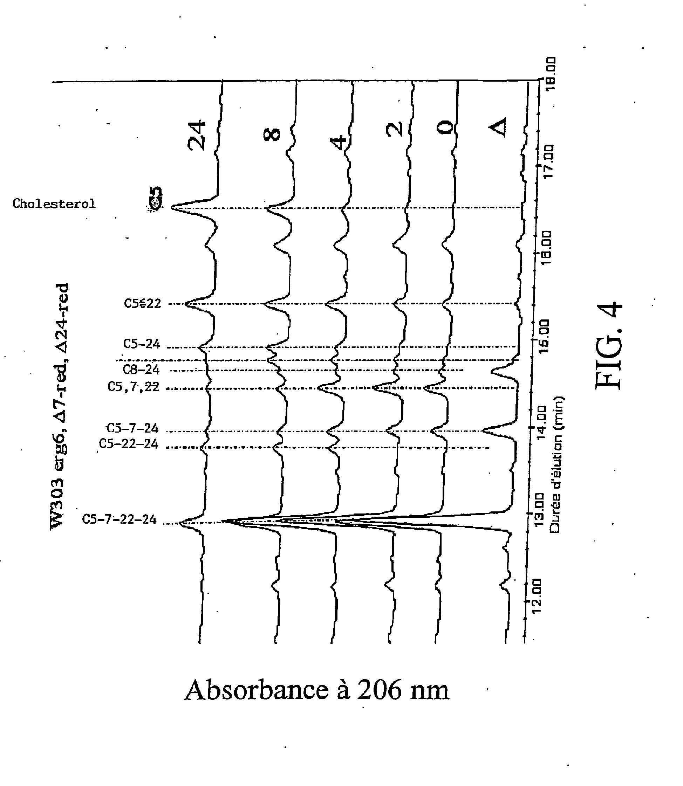

Figure 4. Profil comparé en HPLC avec détection UV à 206 nm des stérols libres de la souche WGIF04 (souche BMA64 disruptée dans le gène *erg6* et exprimant la Δ7-réductase et la Δ24-réductase, Exemple 14) après 0,2,4,8,24 heures d'induction par le galactose. Δ :souche WGIF01 (Exemple 1). Pour la souche WGIF04, les prélèvements sont effectués 0, 2, 4, 8 et 24 h après le basculement de la source de carbone en galactose. Le profil de la souche BMA64 portant la disruption *erg6* (WGIF01) présenté est celui obtenu immédiatement après le basculement en galactose. Ce profil reste pratiquement inchangé au cours de l'induction (0-24 h). Le signal d'absorption à 206 nm correspond à des coefficients d'absorption variables d'un stérol à l'autre. C5.: cholesta 5eneol (cholestérol) ; C5,22: cholesta 5,22 diéneol ; C5, 24: cholesta 5,24 diéneol (desmostérol) ; C8,24: cholesta 8,24 diéneol (zymostérol); C5,7,22: cholesta 5,7,22 triénol; C5.7, 24: cholesta 5,7,24 triénol; C5,22,24: cholesta 5,22,24 triénol; C5,7,22,24: cholesta 5,7,22,24 tetraénol ; lan : lanostérol.

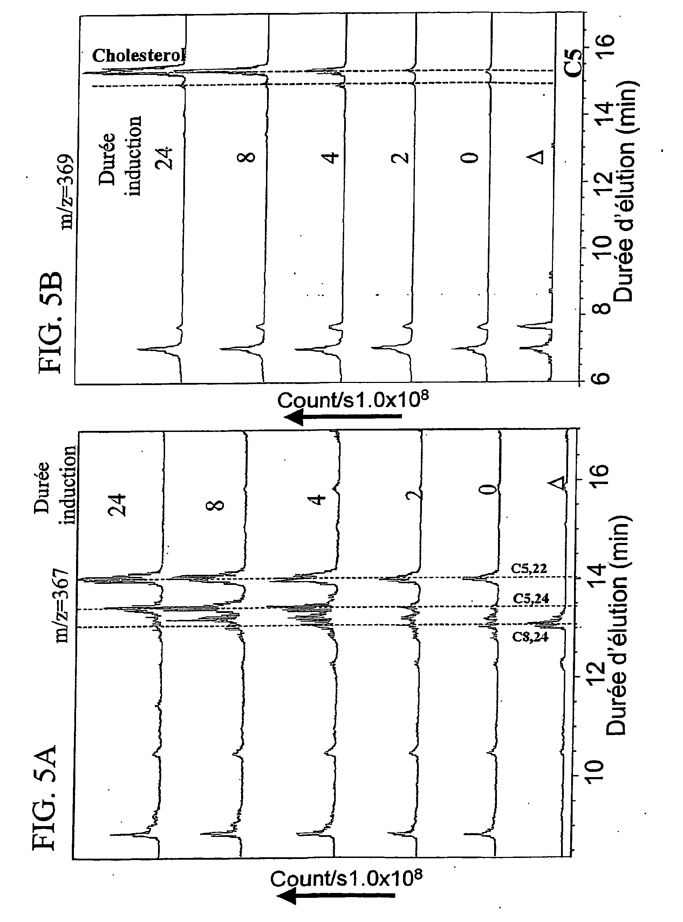

Figure 5. Profil comparé en HPLC avec détection en electrospray avec ionisation positive (spectrométrie de masse) des stérols libres de la souche WGIF04 (Exemple 14) après 0,2,4,8,24 heures d'induction par le galactose. Δ :souche WGIF01. C5 : cholesta 5eneol (cholestérol) ; C5,22: cholesta 5,22 diéneol ; C5, 24: cholesta 5,24 diéneol (desmostérol) ; C8,24: cholesta 8,24 diéneol (zymostérol). Les profils HPLC proviennent du même essais que ceux de la figure 4.
Figure 5A (gauche) : détection à m/z =367, Figure 5B (droite) m/z = 369. Ordonné : nombre d'ions comptés/ seconde. Abscisse : durée d'élution en minutes.

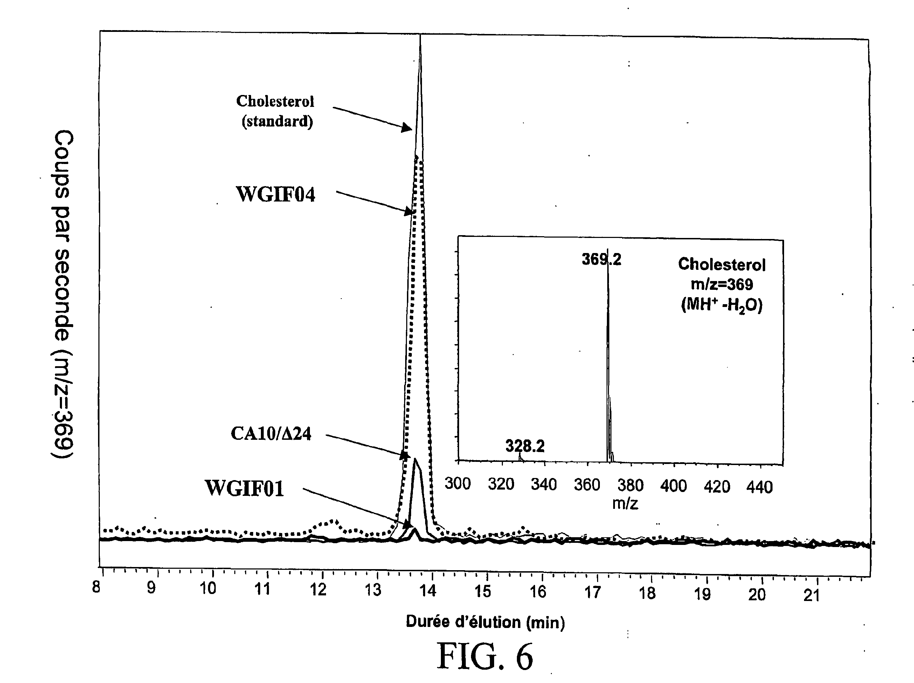

Figure 6. Détail du profil à m /z =369 en HPLC pour les trois souches : WGIF01, CA10 portant le plasmide d'expression pour la delta 24 stérol Réductase et pour WGIF04, le cholestérol est injecté comme standard interne. Les quantités de stérols totaux injectées pour les trois souches correspondent à des extractions faites à partir des quantités identiques de culture mesurées par l'absorbance à 600nm.

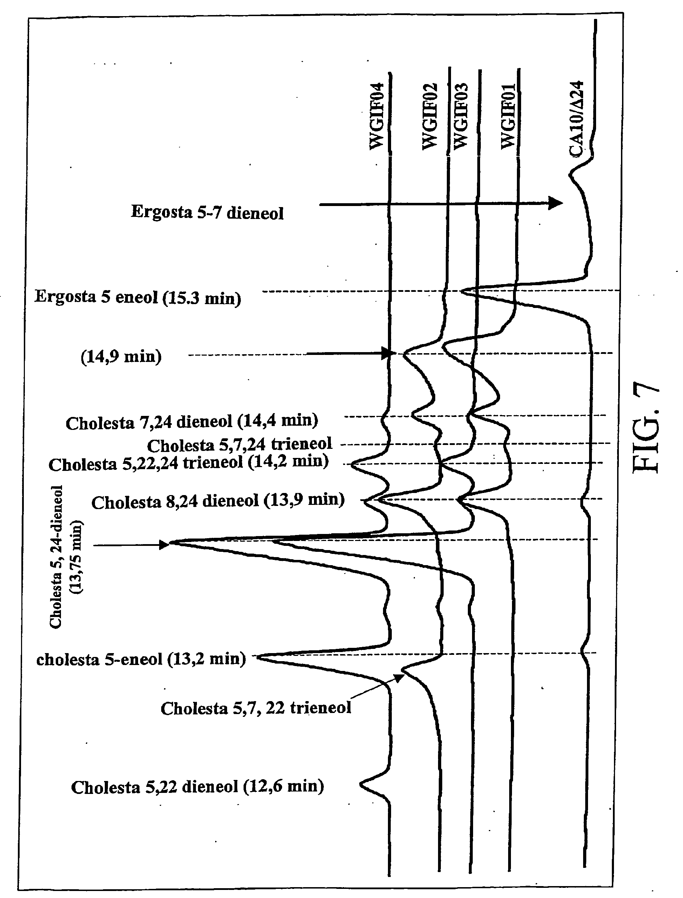

Figure 7 : Profils comparés des stérols totaux (libres et esters) en chromatographie phase gaz des souches WGIF01 (délétion d'erg6), WGIF02 (délétion d'erg6 avec expression de la Δ24-réductase), WGIF03 (délétion d'erg6 avec expression de la Δ7-réductase), WGIF04 (délétion d'erg6 avec expression de la Δ24-réductase et de la Δ7-réductase) et CA10 pYES_Delta24 (fond génétique FY1679, délétion d'erg5 avec expression de la Δ24-réductase, Δ-7 réductase, erg5). Les échelles de réponse (courants d'ionisation de flamme) sont arbitraires. Les profils ne sont à comparer que de manière qualitative d'une souche à l'autre. L'échelle de temps de rétention est néanmoins la même pour toutes les souches (les temps de rétention sont exprimés en minutes). L'identification des stérols est effectuée selon les critères décrits dans la présente demande.

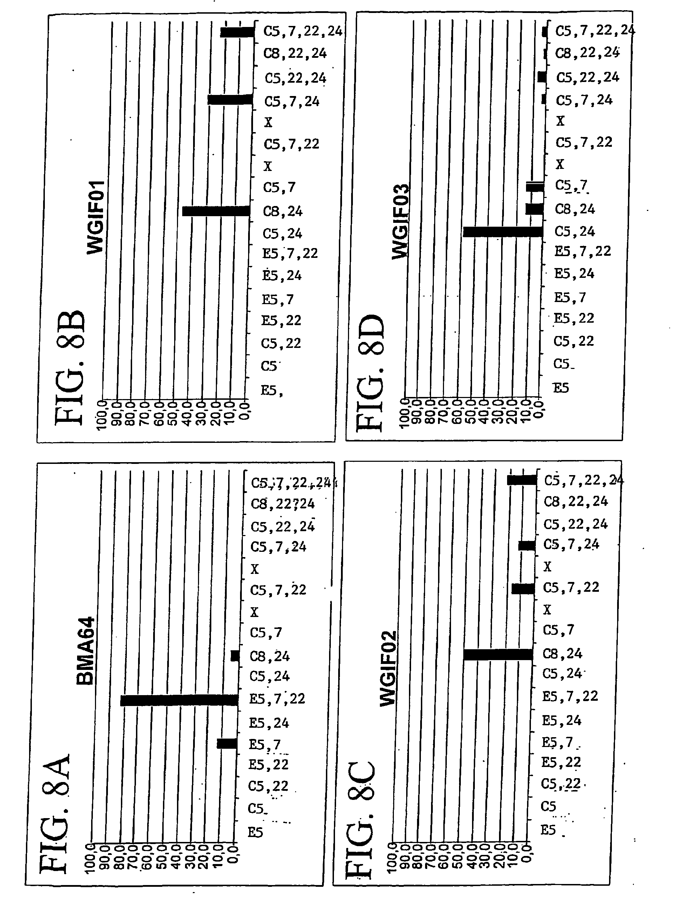

Figure 8. Répartition quantitative des principaux stérols libres dans les souches de levure (BMA64 (Figure 8A), WGIF01 (Figure 8B), WGIF02 (Figure 8C) et WGIF03 (Figure 8D)) évaluée sur la base des spectres UV. La répartition est donnée en % de la totalité des espèces présentées dans la figure et qui sont les seules détectables en quantités appréciables. En absence de standard pour plusieurs des stérols intermédiaires la quantification est effectuée sur la base des spectres UV associés à chacun des pics du chromatogramme HPLC en utilisant les coefficients d'absorption évalués donnés ci dessous (cf. table 1, les coefficients d'absorption sont exprimés en mM par litre et par cm.). Pour ce faire les coefficients d'absorption correspondant aux motifs structuraux insaturés présents dans la structure d'un stérol donné sont recherchés dans la table 1 et éventuellement additionnés (si plusieurs motifs sont présents dans une même molécule) pour fournir une évaluation du coefficient d'extinction de chaque type de stérol. L'évaluation est faite en utilisant les valeurs à 280 nm si au moins un motif absorbant à cette longueur d'onde est présent, à défaut la longueur d'onde 235 nm est utilisée, et à défaut d'absorption à cette dernière, la longueur d'onde 206 nm est utilisée pour évaluer les concentrations de chacun des stérols à partir des signaux d'absorption respectifs en HPLC.

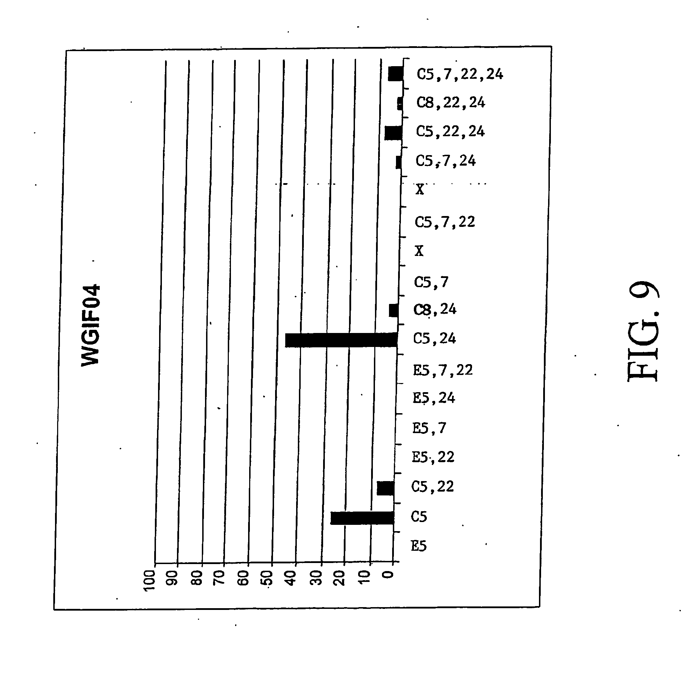

Figure 9. Répartition quantitative des principaux stérols libres dans la souche de levure WGIF4 évaluée sur la base des spectres UV. Les quantifications sont effectuées de la même façon que décrite dans la Figure 8.
Figure 10. Principe du marquage isotopique des stérols par substitution de sources de carbone.

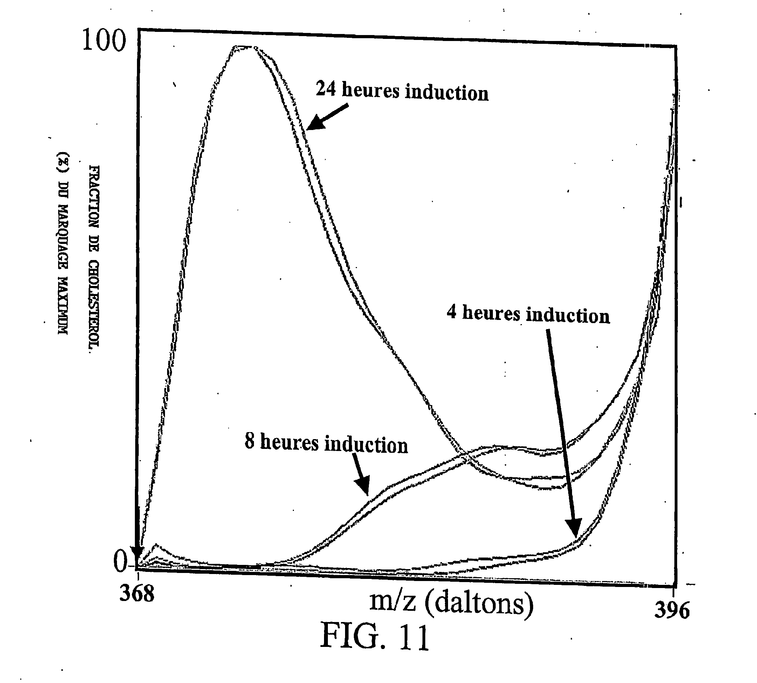

Figure 11. Evaluation des profils de marquage isotopique du cholestérol produit dans la souche WGIF04 après 4, 8, et 24 heures d'induction. Les stérols libres sont extraits et séparés par HPLC comme décrit. Un spectre de masse entre les valeurs de m/z 300 et m/z=450 est acquis toutes les 0,2 secondes durant l'élution. Ces spectres sont alors moyennés sur des fenêtres de 1,8 secondes puis soumis à une régression multilinéaire, en utilisant comme base de régression un ensemble de 24 vecteurs représentant les distributions théoriques de masse du cholestérol marqué pour une incorporation au hasard par des tirages indépendants du carbone 13 à chacune des 27 positions de la molécule avec une probabilité de marquage sur chaque carbone variable entre 0 et 1 selon le vecteur considéré. Les probabilités de marquage des différents vecteurs utilisés comme base sont choisies de telle façon à ce que le coefficient de cross-correlation des distributions de deux vecteurs consécutifs de la base soit de 0,92, la base démarrant avec un vecteur correspondant à une probabilité de présence de 100 % à toutes les positions pour le carbone 12. L'ajustement multilinéaire est effectué sur un critère statistique de moindre carré en annulant les termes non-diagonaux de la matrice des produits des dérivés partielles de la méthode de Gauss (filtrage numérique maximal). Après analyse, les spectres de masse sont alors reconstruits sur la base optimisée. Les courbes représentées sur les figures représentent donc le résultat de la reconstruction filtrée optimale après normalisation de l'amplitude maximale à la valeur 100.
   Pour chaque temps d'induction les deux courbes représentent deux profils indépendants correspondant à des temps d'élution différents de 1.8 secondes et correspondant à des spectres situés dans la zone centrale du pic d'élution du cholestérol. La figure démontre la grande reproductibilité de l'analyse.
Figure 12. Exemple de différentes signatures- isotopiques au niveau de différents stérols ou de différents temps d'induction. Même calcul et représentation que pour la figure 11, mais pour différents stérols et différent temps d'induction.
   La valeur de RT indique la gamme de temps de rétention utilisée pour le calcul (en minute). Les valeurs de cette gamme sont les suivantes :
   Fig. 12 A : RT=12,25-12,42,
   Fig. 12 B : RT=12,2-12,7,
   Fig. 12 C : RT=12,25-12,35,
   Fig. 12 D : RT=13,3-13,6,

   Les temps d'induction sont de 8 ou 24 heures.
   Les valeurs de m/z indiquent la limite gauche et droite des m/z. La valeur la plus faible de m/z pour chaque cadre correspond au m/z pour le stérol totalement constitué de carbone 12.
Figure 13: Profils comparés des stérols totaux (libres et esters) en chromatographie phase gaz des souches YIM59/pIM303 (partie A de la figure) et de la souche YIM59/pIM331 (partie B de la figure) (cf. exemple 18). Les échelles de réponse sont arbitraires. L'échelle de temps de rétention est la même pour les deux souches (les temps de rétention sont exprimés en minutes). L'identification des stérols est effectuée selon les critères décrits dans la présente demande.

La présente invention est illustrée à l'aide des exemples qui suivent, qui doivent être considérés comme illustratifs et non limitatifs.

Les techniques de biologie moléculaire utilisées sont décrites par Ausubel *et al.,* certaines manipulations des levures sont décrites par Adams *et al* (Adams and Holm, 1996).

### EXEMPLE 1 : Construction d'une souche de levure S. cerevisiae interrompue dans le gène erg6 (souche WGIF01) :

La souche de levure *S*. *cerevisiae* WGIF01 dont le gène *ERG6* est interrompu par le gène *TRP1* a été obtenue par transformation de la souche BM64 par un produit de PCR portant un gène fonctionnel *TRP1* bordé par des extrémités homologues au gène *ERG6.*

La souche BM64 (de génotype *MATa; ura3-52; trp1Δ2; leu2-3_112; his3-11; ade2-1; can1-100*) est un dérivé de la souche de levure *S*. *cerevisiae* W303 MATα par délétion complète du gène *TRP1.* La souche BMA64 et la souche W303 MATα sont décrites dans la publication de Baudin-Baillieu et al (Baudin-Baillieu et al., 1997). Pour isoler le gène *TRP1,* le gène *TRP1* du plasmide pFL44 (Bonneaud et al., 1991) a été amplifié en utilisant la *Z-Taq*I (une DNA polymérase DNA dépendante) fournie par la Société Takara (PanVera LLC 501 Charmany Drive Madison, W1 53719 USA). Le couple d'amorce utilisé permet l'amplification par la DNA polymérase du gène *TRP1* bordé de séquences correspondant au gène *ERG6.* La séquence de ces amorces est la suivante :

Le produit de PCR (Polymerase Chain Reaction) ainsi obtenu est purifié par électroélution du fragment correspondant à la taille attendue et utilisé pour transformer la souche BM64 par la technique du chlorure de lithium comme décrit par (Gietz et al., 1995).

Après transformation, les levures traitées sont étalées sur un milieu minimum ne contenant pas de tryptophane (Gietz et al., 1995). On obtient ainsi 41 colonies BM64 transformées prototrophes pour le tryptophane. Ces 41 colonies sont ensuite testées pour trois de leurs propriétés : sensibilité à la nystatine, structure génomique de l'insertion du gène *TRP1,* profil en chromatographie phase gazeuse des stérols totaux qu'elles produisent.

Pour cela, les 41 colonies sont transférées sur un milieu minimum contenant respectivement 10, 20 ou 50µg/ml de nystatine, une dizaine de colonies est capable de croître sur le milieux contenant une dose de 50µg/ml de nystatine. Ces colonies résistantes sont sélectionnées pour vérifier leur structure génique ainsi que leurs compositions en stérols.

L'insertion du gène *TRP1* dans le gène *ERG6* est vérifiée par PCR en utilisant un couple d'oligonucléotides couvrant la jonction entre le gène *TRP1* fonctionnel et le *ERG6* disrupté. Ce couple d'oligonucléotides est le suivant: OERG6trp3 : AGGGCCGAACAAAGCCCCGATCTTC (SEQ ID N° 3) et OERG6trp4 : GGCAAACCGAGGAACTCTTGG (SEQ ID N° 4).

Quelques souches présentent le profil PCR attendu c'est à dire un fragment de 800 paires de base correspondant à la taille attendue pour une insertion de *TRP1* dans *ERG6.*

Dans le but de vérifier que le gène *ERG6* est bien inactivé dans ces souches, une analyse des compositions en stérol de ces souches par la chromatographie en phase gazeuse et par chromatographie liquide haute pression a été réalisée (Duport et al., 2003; Szczebara et al., 2003).

Ces analyses confirment l'absence de synthèse d'ergostérol et l'accumulation de stérols anormaux compatibles avec les perturbations attendues de la voie de biosynthèse dans la souche disruptée.

Une souche a été plus particulièrement sélectionnée et nommée WGIF01.

### EXEMPLE 2 : Construction des souches CA10, CA14 et CA23.

Les souches **CA10** (de génotype: *MATα, rho⁺, GAL2, ura3-52, trp1-*Δ*63, his3-*Δ*200, erg5::HYGRO^{R}, ade2::GAL10*/*CYC1 :: Δ7Réductase ::PGK1, LEU2 ::GAL10*/*CYC1::matADR::PGK1*), **CA14** (de génotype : *MATα, rho⁺, GAL2, ura3-52, trp1-*Δ*63, his3-*Δ*200, erg5::HYGRO^{R} atf2:: G418^{R}, adé2::GAL10*/*CYC1::Δ7Réductase::PGK1, LEU2::GAL10*/*CYC1::matADR::PGK1*)*,* et **CA23** (de génotype : *MATα, rho⁺, GAL2, ura3-52, trp1-*Δ*63, his3-*Δ*200, erg5:: HYGRO^{R}, are1::G418^{R}, are2::HIS3, ade2:: GAL10*/*CYC1::Δ7Réductase::PGK1, LEU2::GAL10*/*CYC1::matADR::PGK1*) ainsi que leurs constructions, sont décrites dans la référence Duport *et al.* dont le contenu technique en ce qui concerne la construction de ces souches est incorporé par référence à la présente demande. Ces souches produisent et contiennent dans leurs membranes des stérols non naturels (comme décrit dans la demande de brevet européen EP 0727 489) et en particulier de l'ergosta-5-énol (campestérol).

Ces trois souches n'expriment pas le produit du gène *ERG5* qui est non fonctionnel par insertion dans sa séquence codante du gène de résistance à l'hygromycine. De plus ces souches expriment le cDNA codant pour la Δ7 réductase de plante (la demande de brevet européen EP 0727 489 décrit notamment le clonage de la Δ7 réductase de la plante *Arabidopsis thaliana*, qui est incorporé par référence à la présente demande, le numéro d'accession GenBank de cette séquence est ATU49398). La souche CA14 est dérivée de la souche CA10 par disruption du gène *ATF2.* Le produit de ce gène conduit à une acétylation de la pregnénolone sur la position 3 (comme il est décrit dans la demande de brevet WO99/40203).

La souche CA23 est une souche dérivée de la souche CA10 par délétion des gènes *ARE1* et *ARE2,* les deux protéines Are1p et Are2p sont responsables de l'estérification de l'ergostérol (Sturley, 2000) et éventuellement du cholestérol car ils sont homologues à l'enzyme responsable de l'estérification du cholestérol chez les mammifères (ACAT).

### EXEMPLE 3 : Construction du plasmide d'expression de la Δ24-25 réductase d'origine humaine (plasmide pYES_Delta24)

La construction de ce plasmide a été décrite par Waterham *et al*. 2001. La construction a consisté à mettre le cDNA codant pour la Delta 24 stérol réductase sous le contrôle du promoteur pGAL1 et du terminateur tCYC1 dans le vecteur pYES2 (Invitrogen SARL, Cergy Pontoise, France). Ce plasmide est un plasmide navette *E.coli*/ *S cerevisiae* et contient une origine de réplication 2micron et un gène *URA3*, ceci permettant sa réplication dans la levure et des facilités de sélection des levures transformées par ce plasmide.

Le promoteur GAL1 est de plus inductible par le galactose.

### EXEMPLE 4 :Construction du plasmide pAG1 exprimant la Δ7-réductase d' A. thaliana.

Un plasmide a été construit spécifiquement pour l'expression de la Delta 7 Réductase de *A. thaliana* sur un vecteur monocopie. Le plasmide pAM1 a été utilisé pour cette construction. Ce plasmide dont la construction est décrite dans la demande PCT WO02/061109 (cf. exemple 9.b de ladite demande, qui est incorporé par référence à la présente demande) est un plasmide navette *E.coli*/ *S. cerevisiae* basé sur une séquence de réplication autonome et un centromére (ARS CEN). Le marqueur de sélection est le gène *ADE2.* Ce plasmide est compatible, et peut donc se répliquer en même temps qu'un plasmide basé sur une origine de réplication 2 micron. Ce plasmide possède en particulier un site unique *Not*I permettant de cloner les cassettes d'expression tel que décrit dans la demande PCT ci-dessus.

Ce site a été utilisé pour cloner une cassette d'expression de la Delta7-réductase *d'A. thaliana* provenant de la souche CA10. En effet, cette cassette d'expression est très efficace et permet à la souche CA10 qui est de plus disruptée pour *ERG5*, de produire du campestérol (ergosta-5-énol) comme stérol majoritaire (Duport et al., 1998). Le fragment d'ADN génomique de la souche CA10 contenant le gène de la delta7-réductase est amplifié à l'aide des amorces suivantes :
OSA72 5' (TATATAGCGGCCGCTTTCGCTGATTAATTACCCCAG) 3' (SEQ ID N° 5)
OSA 77 5' (TATATAGCGGCCGCGAGAAGTGACGCAAGCATCA) 3' (SEQ ID N° 6).

L'amplification est réalisée sur de l'ADN génomique de la souche CA10 préparé par la technique rapide d'extraction au phenol/chloroforme comme décrit par Adams *et al (Adams and Holm, 1996).*

Cinquante nanogrammes d'ADN génomique de CA10 sont utilisés comme matrice pour amplification à l'aide des amorces OSA72 et OSA77. La Taq DNA polymérase et les conditions enzymatiques provenaient de la société Stratagene. Les conditions d'amplification étaient les suivantes. Dénaturation initiale de 5 min à 95°C, puis ensuite, trente cycles sont réalisés consistant en une dénaturation de 30s à 95°C, une hybridation de 30s à 50°C puis une élongation de 1 min à 72°C. La réaction est terminée par une extension finale de 10 min à 72°C.

Le fragment de PCR est ensuite digéré par l'enzyme *Not*I et purifié sur gel d'agarose puis cloné classiquement au niveau du site unique *Not*I du plasmide pAM1. Le plasmide ainsi obtenu a été nommé pAG1.

Il s'agit d'un vecteur monocopie permettant l'expression de la Delta7 Réductase de *A*. *thaliana* chez la levure, le gène de la Delta7 Réductase étant placé sous le contrôle du promoteur GAL10/CYC1 (Lecain et al., 1996).

### EXEMPLE 5 : Extraction des stérols libres et estérifiés chez la levure pour les analyses :

### 1) Conditions d'extraction des stérols libres et estérifiés chez la levure. (procédure1)

### a) Conditions d'extraction des stérols libres :

Le culot cellulaire est lavé deux fois avec 500µl d'eau désionisée et filtrée, dans un tube en verre.

Les cellules sont ensuite resuspendues dans 500µl d'eau contenant des billes de verre de 0,5 mm correspondant à 150µl de liquide dans le tube.

Une extraction est réalisée deux fois avec 2ml de 1,2 dichloroéthane avec une agitation forte sur un vortex pendant 10 minutes. Après la première extraction, le mélange cellules, billes de verre, solvant est centrifugé pendant 5 minutes à 1500g dans le but de séparer les deux phases.

Les deux fractions organiques issues des deux extractions successives sont rassemblées et séchées sous un courant d'azote pendant quelques heures. L'extrait stérolique est suspendu dans 100µl d'acétonitrile pour son analyse par chromatographie liquide haute performance (HPLC) (Szczebara et al., 2003) ou dans 100µl d'hexane pour les analyses par chromatographie en phase gazeuse (GC) (Duport et al., 2003).

### b) Conditions d'extraction des stérols totaux : Saponification et extraction des stérols estérifiés, analyse qualitative procédure 1 :

Le culot cellulaire est resuspendu dans 500µl d'eau purifiée. A cette suspension on ajoute 2ml d'hydroxyde de potassium KOH à 10% dans du méthanol. Le mélange est chauffé pendant une heure à 60°C dans des tubes fermées. Après l'incubation et une fois que les tubes sont revenus à température ambiante, le mélange est extrait trois fois avec 2ml d'hexane. Entre chaque extraction, les deux phases sont séparées par une centrifugation de 5min à 1500g. Après chaque extraction, la phase organique est transférée dans un nouveau tube, puis les trois phases organiques sont rassemblées puis séchées sous un flux d'azote.

Le résidu stérolique est resuspendu dans 100µl d'acétonitrile 100% pour son analyse par chromatographie liquide haute performance (HPLC) (Szczebara et al., 2003) ou dans 100µl d'hexane pour les analyses par chromatographie en phase gazeuse (GC) (Duport et al., 2003).

### 2) Conditions d'extraction des stérols libres et estérifiés chez la levure pour analyse qualitative (procédure2).

Les souches sont cultivées en milieu riche (10g de bactopeptone par litre et . 10 g d'extraits de levure par litre) avec 2% de glucose comme source de carbone afin d'obtenir 500mg de cellules lyophilisées. Ces cellules séchées sont reprises dans 3ml de méthanol (100%) contenant 1g de KOH et une trace de pyrogallol puis le mélange est incubé pendant 45 minutes à 90°C. Après retour à température ambiante, les stérols sont extraits avec 5ml d'hexane. La phase organique est séparée en trois échantillons de même volume et séchée sous un courant d'air. Deux des échantillons des stérols extraits sont repris dans 100µl d'hexane pour les analyses par chromatographie en phase gazeuse (GC) et chromatographie en phase gazeuse couplée à la spectrométrie de masse GC/MS et le troisième échantillon est repris dans 150µl de méthanol pour les études par chromatographie liquide haute performance (HPLC).

### EXEMPLE 6 : Analyse des stérols libres et estérifiés chez la levure par chromatographie en phase gazeuse (GC)

### 1) Chromatographie en phase gazeuse (GC) avec détection FID (ionisation de flamme)

L'extrait stérolique (libre ou total) suspendu dans l'hexane est préparé selon la procédure 1 (cf. exemple 5 1) a) et b)). Un témoin d'injection est ajouté au mélange de stérol, en général le cholestérol à une concentration de 10 à 50ng/*µ*l. On injecte ensuite de 1 à 3*µ*l d'échantillons sur un appareil de chromatographie en phase gazeuse dans les conditions suivantes. 1 à 3*µ*l ont été injectés sur une colonne Alltech de type SE30 (Référence de la colonne : 30mX0.32mm IDX 0.25*µ*m).Le gaz vecteur est l'hélium. Le rapport de Split est entre 50 et 80. La pression en tête de colonne est de 30 psi. L'injecteur est réglé à 280°C. La température initial de la colonne est de 130°C pendant 0.5 minute. Elle monte à 230°C à raison de 40°C/min, puis de 230°C à 280°C à raison de 3°C/min. La colonne est ensuite maintenue à 290°C. La température du détecteur est de 310°C.

### 2) Chromatographie en phase gazeuse (GC) avec détection FID (ionisation de flamme) couplée à la spectrométrie de masse (GC/MS).

L'extrait stérolique total suspendu dans l'hexane est préparé selon la procédure 2. La GC utilisée est équipée d'un injecteur classique « split split less » avec une colonne classique DB5 d'une longueur de 30 mètres et d'un diamètre de 0,25mm.

L'injection est réalisée à 230°C avec comme gaz vecteur, l'hélium au débit de 2ml/mn. La colonne passe de 130 à 290°C en 4 étapes. La colonne est maintenue à 130°C avant l'injection puis monte à 230°C avec une rampe de 40°C/mn puis de 230°C à 280°C avec une rampe de 3°C/mn puis de 280°C à 290°C avec une rampe de 30°C/min. La colonne reste pendant 5 minutes à 290°C.

En sortie de la colonne de chromatographie en phase gazeuse, les molécules sont ensuite analysées par spectrométrie de masse par vaporisation dans une chambre d'ionisation telle que celle d'un appareil de type Turbo Mass de chez Perkin Elmer. Les molécules sont fragmentées par un faisceau d'électrons de haute énergie. Les différents fragments sont ensuite séparés sur un filtre quadrupolaire puis détectés sur un détecteur d'ions. A chaque masse localisée sur le graphe de courant ionique correspond un spectre de masse qui regroupe les masses de tous les produits de fragmentation de l'ion M+. Ce spectre de masse obtenu à un temps de rétention donné sur la colonne est comparé à des bibliothèques de produits fragmentés ainsi qu'à celles décrites pour les stérols par Quail and Kelly. (Methods in Molecular Biology Vol53 Yeast Protocols Edited by Evans; M, Quail and S, Kelly "The Extraction and Analysis of Sterols from Yeast" pp 123-131 (1996)).

De cette façon, il a pu être mis en évidence l'effet de la délétion du gène *ERG6* dans la souche WGIF01 et notamment l'absence d'ergosta 8, 24(28) dienol et la présence de stérol de type cholesta ayant la double liaison en 24(25).

### EXEMPLE 7 : Analyse des stérols libres et estérifiés chez la levure par chromatographie liquide haute performance (HPLC) avec détection UV ou détection par spectrométrie de masse.

### 1) Analyse par HPLC détection UV :

Dix à 30*µ*l de l'extrait stérolique (suspendu dans l'acétonitrile ou le méthanol et préparé selon la procédure 1 ou 2 (cf. exemple 5)) sont injectés sur une colonne de type X terra RP18, 4,6X100mm (Waters, Milford, MA01757 USA).

La séparation s'effectue sur un gradient composé d'eau contenant 0.02 % de TFA (acide trifluoroacetique) (tampon A) et d'acétonitrile pur (tampon B). La colonne est maintenue à 60°C au cours de l'analyse.

L'appareil HPLC utilisé est de type « Waters 600 E System Controller » (Waters, Milford, MA01757 USA). La détection UV a été réalisée sur un détecteur à barrette de diodes couvrant les longueurs d'onde de 206 à 350 nm. La colonne a été équilibrée avec un tampon à 20% (v/v) de tampon A (acétonitrile) et 80 % de tampon B (eau contenant 0.02 % de TFA (acide trifluoroacetique)). Un gradient linéaire est effectué à partir d'une solution contenant 50 % de tampon A et 50 % de tampon B. Au bout de 10 min, la composition du tampon d'élution est de 25% de tampon A pour 75 % de tampon B. Un nouveau gradient linéaire est alors appliqué de façon à ce qu'à 30 min le gradient atteigne la valeur de 100 % en tampon B. Cette valeur est maintenue pendant 5 minutes pour nettoyer la colonne.

### 2) Analyse par HPLC détection par spectrométrie de masse (HPLC/MS):

Dans le cas d'une analyse par spectrométrie de masse, l'échantillon est maintenu à 30°C, et la colonne est maintenue à 60°C au cours de l'analyse. L'appareil HPLC utilisé est du type « Alliance HT Waters 2790 », couplé à un détecteur de masse « Waters MicroMass ZQ ». A la différence de la méthode de détection précédente le tampon d'élution A ne contient pas de TFA mais les deux tampons A et B contiennent 0.01 % (v/v) d'acide formique.

La colonne a été équilibrée avec un tampon contenant 80% de tampon A' (eau contenant 0.01% (v/v) d'acide formique) et 20% de tampon B' (acétonitrile contenant 0.01 % (v/v) d'acide formique).

L'injection démarre avec un tampon contenant 50% de ces deux tampons. Un gradient linéaire à deux rampes est effectué à partir d'une solution contenant 50 % de tampon A' et 50 % de tampon B'.

Au bout de 10 min, la composition du tampon d'élution est de 25% de tampon A' pour 75 % de tampon B'. La rampe du gradient est ensuite modifiée pour atteindre 12,5% de tampon A' et 87,5% de tampon B' après 25 minutes d'analyse puis 100 % de tampon B à 30 minutes. Cette valeur est maintenue pendant 5 minutes pour régénérer la colonne.

Le détecteur de masse « Waters MicroMass ZQ » est réglé pour balayer en ionisation positive electrospray les valeurs de m/z comprise entre 295 à 450. Un mode d'acquisition « continum » est choisi pour le balayage. Par ailleurs une extraction de signal en mode « SIR » est effectuée en parallèle à toutes les masse attendues en abondance isotopique naturelle pour les stérols à analyser. Le détecteur est réglé de manière à résoudre totalement sans interférence des molécules différant de 1 unité de m/z. La totalité des acquisitions est paramétrée de façon à ce que la durée totale d'acquisition correspondant au balayage et au temps total d'acquisition de l'ensemble des SIR reste inférieure à 2 secondes.

### EXEMPLE 8: Culture des souches de levures pour l'analyse de leur contenu en stérol avec ou sans marquage ¹³C :

Les souches à analyser ont été cultivées dans un volume de 50ml de milieu Kappeli (Kappeli et al., 1985) contenant 2% de D-glucose normal ou de D-glucose-U-¹³C₆ (pour les expériences de marquage, cf. figure 10).

La densité optique de la culture de départ est de 0.1 à 600nm. Cette culture est incubée 72 heures à une température de 30°C sous agitation à 200 tours/ min.

Les cellules sont ensuite récupérées par centrifugation du milieu à 600 g pendant 10 minutes. Le culot cellulaire est ensuite analysé directement par les techniques d'analyse présentées dans l'exemple 5 (pour les études ne nécessitant pas d'induction au galactose).

Toutefois, pour les études de cinétique d'induction de l'expression de la delta 7-réductase et la delta 24-réductase (souches transformées par le plasmide pYES_Delta24 et/ou pAG1), le culot est resuspendu dans 50ml de milieu Kappeli frais contenant 2% galactose (non marqué au carbone ¹³C).

Cette culture est incubée à une température de 30°C sous agitation à 200 tours/ min. Dix ml de culture sont récupérés après 0 heure, 2 heures, 4 heures, 8 heures et 24 heures de cultures.

Ces échantillons de culture sont centrifugés à 800 g pendant 10 minutes, le culot cellulaire est congelé et conservé à -20°C avant extraction stérolique par les méthodes décrites dans l'exemple 5.

### EXEMPLE 9: Identification des stérols présents dans les souches analysées :

L'identification des stérols est basée sur la combinaison des principes suivants :
- Comparaison du comportement en GC, HPLC, GC/MS et HPLC/MS avec des standards authentiques dans le cas du campestérol (ergosta 5,eneol), de l'ergostérol (ergosta 5,7,22 triénol), du cholestérol (cholesta 5-eneol), du desmostérol (cholesta 5, 24 diéneol), du cholesta 5,22 diénéol et du zymostérol (cholesta 8,24 diéneol).
- Analyse du spectre d'absorption en HPLC et détection UV à barrette de diodes (cf. exemple 7-1)) :

Cette méthode permet d'identifier sans ambiguïté sur la base spectrale cinq classes de stérols : 1) classe SA1 : pas de système diénique conjugué, 2) classe SA2 : présence d'un système 5,7-diénique ; 3) classe SA3 : présence d'un système 22,24(25) diénique ; 4) classe SA4 : présence d'un système 8,14 diénique ; 5) classe SA5 : présence d'un système 22, 24(28) diénique. Les classes SA3 et SA5 ne peuvent coexister pour des raisons structurales. Les classes SA2 et SA4 ne peuvent coexister pour des raisons de biosynthèse, la classe SA2 peut être associée à des motifs structuraux de classes SA1, SA3, SA5 pour former des spectres composites additifs.
- Analyse des temps de rétention en GC et en HPLC sur la base d'une additivité approximative des déplacements de temps de rétention associés à chaque type d'insaturation et à la présence d'un squelette de type ergosta ou cholesta. Ce critère n'étant pas absolu, il est utilisé comme aide à l'identification et pour lever les ambiguïtés mais présente un risque d'erreur s'il est utilisé seul. Il n'est donc utilisé qu'en combinaison avec les autres critères.
- Analyse en GC/MS (cf. exemple 6-2)) qui fournit la masse moléculaire et un profil de fragmentation pouvant être comparé à des bibliothèques de spectres.
- Analyse en HPLC/MS (cf exemple 7-2)) en electrospray qui fournit, dans le cas des 3-hydroxystérols, un signal principal à la masse moléculaire -17 (protonation (+1) et perte d'une molécule d'eau (-18))
- Analyse avec l'ensemble des systèmes ci-dessus de la composition en stérols de différentes souches de levures de référence disruptées à différents points de la biosynthèse.
- Analyse des variations de la composition en stérol lors de la complémentation par différentes enzymes de biosynthèse et de la cinétique de cette complémentation lors de l'induction de cette complémentation.
- Analyse du profil de marquage des différents stérols par l'isotope 13 du carbone.
- Analyse du spectre UV des stérols séparés par HPLC à un temps de rétention donné.

Les deux doubles liaisons 5, 7 conjuguées présentent un spectre typique avec deux pics d'absorption entre 265 et 280 nm tandis que les deux double liaison 22, 24 conjuguées présentent un pic d'absorption à 235nm. La dernière double liaison conjuguée en 8, 14 est identifiable par un pic d'absorption à 245 nm.

### EXEMPLE 10: Identification des stérols présents dans la souche BMA64 :

La souche BMA64 est cultivée dans un volume de 50ml de milieu Kappeli contenant 2% de D-glucose pour l'analyse quantitative et comparative des stérols. La densité optique de la culture de départ est de 0.1 à 600nm. Cette culture est incubée 72 heures à une température de 30°C sous agitation à 200 tours/ min.

Les cellules sont ensuite récupérées par centrifugation du milieu à 600 g pendant 10 minutes, le culot cellulaire est analysé par les techniques présentées dans l'exemple 5. Les différentes analyses décrites ont permis d'identifier les stérols produits par cette souche.

On a ainsi déterminé que cette souche accumule plus de 80% de ses stérols libres sous forme d'ergostérol (ergosta 5,7,22 triénol) (cf. Figure 8). Deux autres stérols minoritaires détectables sont produits par cette souche, il s'agit de l'ergosta 5,7 diéneol (substrat du produit du gène ERG5) (12%) et le zymostérol (ergosta 8,24 diéneol) (5%). Aucune trace de cholestérol n'est détectable (la limite de détection de la méthode est d'environ 0.5 % des stérols observables). De faibles quantités de lanostérol sont également détectables (uniquement au niveau des analyses des stérols totaux).

### EXEMPLE 11 : Identification des stérols présents dans la souche WGIF01 :

La souche WGIF01 (cf. exemple 1) a été cultivée dans un volume de 50ml de milieu Kappeli (Kappeli et al., 1985) contenant 2% de D-glucose pour l'analyse quantitative et comparative des stérols.

La densité optique de la culture de départ est de 0.1 à 600nm. Cette culture est incubée 72 heures à une température de 30°C sous agitation à 200 tours/ min. Les cellules sont ensuite récupérées par centrifugation du milieu à 600 g pendant 10 minutes, le culot cellulaire est analysé par les techniques présentées dans l'exemple 5. Les différentes analyses décrites ont permis d'identifier les stérols produits par cette souche.

La recherche dans le chromatogramme d'ergosta 5,7,22 triénol (ergostérol) ou de ergosta 5,7 diéneol est négative (moins de 0,5% de la valeur obtenue chez BMA64) en HPLC couplée à la spectrométrie de masse. Au niveau des stérols libres, la souche accumule pour 50% du total du zymostérol (cholesta 8,24 diéneol), substrat du produit du gène ERG6, et pour 30 et 20 % respectivement, du cholesta 5,7,24 trienol et du cholesta 5,7,22,24 tetraenol résultant probablement d'un mécanisme de synthèse identique à celui conduisant à l'ergosta 5,7 dienol et à l'ergosta 5,7,22 trienol chez la souche parentale (cf. figure 3 et 8). Ceci montre bien que la voie de biosynthèse est bloquée au niveau de *erg6* puisque l'enzyme ERG6p (S-adenosylmethionine delta24 stérol C-methyl transférase) transforme le cholesta 8,24 (25) diéneol en ergosta 8, 24(28) diéneol (cf. figure 2). Cette accumulation indique clairement que la souche WGIF01 ne possède pas de copie fonctionnelle du gène *erg6.* Les résultats indiquent également que la voie de biosynthèse normale de l'ergostérol chez la levure et en particulier la stérol 8,7-isomerase, la stérol 5-desaturase et la stérol delta 22-desaturase, sont capables de convertir les substrats de type cholesta- avec une activité qui reste importante.

### EXEMPLE 12 : Construction de la souche WGIF02 et identification des stérols présents dans cette souche :

La souche WGIF02 a été obtenue par transformation de la souche WGIF01 par le plasmide pYES2 portant la cassette d'expression de la A24 Réductase (pYES_Delta24, cf. exemple 3). Les clones ont été sélectionnés sur un milieu dépourvu en uracile et la présence et l'expression du cDNA de Δ24 réductase est vérifiée en analysant les stérols de ces transformants par la procédure 1 (cf. exemple 5-1)).

Un clone nommé WGIF02 a été sélectionné car il possédait un profil stérolique différent de la souche WGIF01, de plus le stérol supplémentaire avait un temps de rétention proche de celui du cholestérol (cf. figure 7). La souche WGIF02 est cultivée dans un volume de 50ml de milieu Kappeli (Kappeli et al., 1985) contenant 2% de D-glucose pour l'analyse quantitative et comparative des stérols.

La densité optique de la culture de départ est de 0.1 à 600nm. Cette culture est incubée 72 heures à une température de 30°C sous agitation à 200 tours/ min.

Les cellules sont ensuite récupérées par centrifugation du milieu à 600 g pendant 10 minutes, le culot cellulaire est analysé par les techniques présentées dans l'exemple 5. Les différentes analyses décrites ont permis d'identifier les stérols produits par cette souche.

Les deux profils d'extraits stéroliques de la souche WGIF01 et de la souche WGIF02 sont semblables sauf à l'apparition d'un nouveau pic identifié par sa masse, son temps de rétention et ses doubles liaisons conjuguées comme le cholesta 5,7,22 trieneol (Figures 2, 3 et 7). La présence de ce composé indique la présence attendue d'une activité 24-25 stérol réductase sur la double liaison en 24(25) du cholesta 5, 7, 22, 24 (25) tétraenol. De plus, la quantité de cholesta 5, 7 24 trienol est diminuée avec apparition du cholesta 5, 7, 22 trienol dans la souche WGIF02 (figures 7 et 8) . L'activité de l'enzyme est mise en évidence par la conversion du cholesta 5,7,24 trienol représentant 30% dans la souche WGIF01 et seulement 12% dans WGIF02, la différence soit 18% se retrouvant intégralement sous forme de cholesta 5,7,22 trienol dans la souche WGIF02. Il s'agit là d'un résultat inattendu dans la mesure ou le produit de conversion du cholesta 5,7,24 par la delta 24-réductase est le cholesta 5,7, qui est absent dans WGIF02 donc quantitativement convertit en cholesta 5,7,22. Ceci met en évidence un autre résultat inattendu, à savoir que le cholesta 5,7 est un substrat de la stérol 22-desaturase alors que le cholesta 5,7,24 est d'après le profil stérolique de la souche WGIF01 un mauvais substrat.

### EXEMPLE 13 : Construction de la souche WGIF03 et identification des stérols présents dans cette souche :

La souche WGIF03 a été obtenue par transformation de la souche WGIF01 par le plasmide pAG1. Ce plasmide navette entre *E.coli* et *S. cerevisiae* porte une casssette d'expression pour la Δ7 réductase dont le cDNA correspondant se trouve sous contrôle du promoteur GAL10/CYC1. La souche WGIF01 a été transformée par la technique du chlorure de lithium et les transformants ont été sélectionnés sur milieu ne contenant pas d'adénine. L'expression de la Delta7 réductase a été vérifiée par l'apparition dans le profil stérolique des bons clones de cholesta 5,24(25) dienol. Un clone répondant à ces critères a été plus particulièrement sélectionné et nommé WGIF03.

La souche WGIF03 a été cultivée dans un volume de 50ml de milieu Kappeli (Kappeli et al., 1985) contenant 2% de D-glucose pour l'analyse quantitative et comparative des stérols.

La densité optique de la culture de départ est de 0.1 à 600nm. Cette culture est incubée 72 heures à une température de 30°C sous agitation à 200 tours/min. Les cellules sont ensuite récupérées par centrifugation du milieu à 600 g pendant 10 minutes, le culot cellulaire est analysé par les techniques présentées dans l'exemple 5. Les différentes analyses décrites ont permis d'identifier les stérols produits par cette souche.

L'expression de la delta7 stérol réductase dans la souche WGEF01 ( pour donner la souche WGIF03), contrairement à l'expression de la delta24 stérol réductase, conduit à un profond changement du profil stérolique de la souche avec disparition presque total des cholesta 5,7, 22, 24 tetraenol, des cholesta 7, 24 dieneol et des cholesta 8,24 dienol.

Cette activité est également marquée par l'apparition d'un pic majoritaire identifié comme décrit précédemment comme le cholesta 5, 24 dieneol or desmostérol. Les quantités de cholesta 8, 24 dieneol passent de 12 à 48%. Le cholesta 5, 7, 24 trieneol détecté passe de 30 à 3% et le cholesta 5, 7, 22, 24 tétraeneol detecté de 23 à 4%, respectivement pour les souches WGIF01 et WGIF03. Ces observations indiquent, de manière inattendue, que la stérol delta 7-réductase réduit les cholesta 5,7 dienol de manière presque indépendante de la nature des insaturations portées par la chaîne latérale des stérols. Ce résultat est opposé à celui observé avec la stérol delta 24-réductase. De manière inattendue, l'expression de la stérol delta 7-réductase conduit également à l'accumulation (12 %) d'une molécule co-migrant avec le cholesta 5,7 diéneol. Il semble néanmoins peu probable bien que non exclus que cette molécule soit du cholesta 5,7 diéneol dont le niveau théorique devrait décroître et non augmenter dans ces conditions. L'apparition de faible quantité de cholesta 5,22,24 trienol (8%) est aussi d'intérêt. Ce dernier stérol est le produit attendu de l'action de la stérol 22-desaturase sur le cholesta 5,24 dienol stérol majoritaire (60%) dans la souche WGIF03 (résultant de la réduction du cholesta 5,7,24 trienol par la stérol delta 7-réductase). La faible accumulation de cholesta 5,22,24 trienol indique de manière inattendu que le cholesta 5,24 dienol n'est pas un bon substrat de la stérol 22-desaturase. Grâce aux résultats obtenus dans la souche WGIF02 (cf exemple 12), il peut être déduit que la présence d'une insaturation en 24 (cholesta 5, 24 dienol ou cholesta 5,7,24 trienol) rend les stérols difficilement métabolisable par la stérol 22-desaturase. L'action de l'enzyme ERG6p en transformant les cholesta insaturés en 24 en ergosta, conduit donc à transformer de mauvais substrats du gène ERG5 (22-desaturase) en bons substrats.

### EXEMPLE 14 : Construction de la souche WGIF04 et identification des stérols présents dans cette souche :

La souche WGIF04 a été obtenue par transformation de la souche WGIF02 par le plasmide pAG1 par la technique du chlorure de lithium et les transformants ont été sélectionnés sur milieu ne contenant pas d'adénine ni d'uracile. Les bons transformants ont alors été confirmés sur la base de la détection de l'accumulation de cholestérol. Un clone répondant à ces critères a été plus particulièrement sélectionné et nommé WGIF04. Un échantillon de la souche WGIF04 a été déposé auprès de la Collection Nationale de Cultures de Microorganismes (CNCM) Institut Pasteur, 25, rue du Docteur Roux 75724 Paris Cedex 15, France, le 22 avril 2004 sous le numéro d'enregistrement I-3203.

Des souches indistinguables de WGIF04 peuvent être aussi obtenues en transformant la souche WGIF03 par pYES_Delta24 et en appliquant la même sélection.

La souche WGIF04 a été cultivée dans un volume de 50ml de milieu Kappeli contenant 2% de D-glucose pour l'analyse quantitative et comparative des stérols. La densité optique de la culture de départ est de 0.1 à 600nm. Cette culture est incubée 72 heures à une température de 30°C sous agitation à 200 tours/ min. Les cellules sont ensuite récupérées par centrifugation du milieu à 600 g pendant 10 minutes, le culot cellulaire est analysé par les techniques présentées dans l'exemple 5. Les différentes analyses décrites ont permis d'identifier les stérols produits par cette souche.

Le cholestérol représente 25 % des stérols libres de la souche WGIF04 (cf. figure 9). La formation de cholestérol dans cette souche est indépendamment démontrée en GC et en HPLC par la co-migration avec un standard authentique et par confirmation à la fois en GC/MS et en HPLC/MS. Le cholestérol est non-détectable (< 0,5% des stérols totaux) dans toutes les souches qui n'expriment pas simultanément la delta 7-réductase et la delta 24-réductase.

Les souches qui ne sont pas disruptées pour le gène *erg6* peuvent produire du cholestérol, toutefois, celui-ci représente moins de 5 % des stérols libres totaux. Ainsi, il a pu être construit la souche BMA64-pYES_Delta24-pAG1 obtenue à partir de BMA64 co-transformée par pYES_Delta24 et pAG1. Cette souche produit du cholestérol, ce dernier représentant quelques % des stérols totaux.

La souche CA10 a été transformée par le plasmide pYES_Delta24. Cette souche produit également du cholestérol, ce dernier représentant quelques % des stérols totaux.

Il a pu être démontré par ailleurs que la formation du cholestérol nécessite bien l'induction des promoteurs de la delta 7-réductase et la delta 24-réductase (cf. figures 4 et 5). Les souches contenant ces gènes ne produisent pas de cholestérol en absence d'induction, la figure 5 indique que le niveau maximal de cholestérol est atteint après environ 24 h d'induction. Parallèlement à la formation de cholestérol (cholesta 5-eneol) on observe également la formation de cholesta 5,22 diéneol. L'analyse de la figure 4 et de la figure 5 indique que la formation de ce dernier composé se produit plus rapidement après l'induction que la formation de cholestérol et commence même avant l'induction (cf. figure 5A : m/z=367). Néanmoins ce composé est totalement absent si la souche ne porte pas les deux plasmides pAG1 et pYES_Delta24. La formation de 22-dehydrocholestérol est donc un processus plus rapide que celui de formation du cholestérol mais ce processus implique un précurseur qui disparaît rapidement après induction laissant place à la formation de cholestérol. Le cholestérol peut être formé à partir de cholesta 5,24 via la Δ 24-réductase ou à partir de cholesta 5,7 via la Δ7-réductase. Or il a été montré que le cholesta 5,7 dienol ne peut s'accumuler du fait de sa conversion immédiate en cholesta 5,7,22 trienol. La source du cholestérol est donc le cholesta 5,24 dienol qui est absent au moment de l'induction et s'accumule vers 4 à 8 heures d'induction avant de diminuer vers 24h (figure 5). Ceci explique l'apparition tardive du cholestérol puisque la synthèse préalable de cholesta 5,24 diéneol est requise. A l'opposé, les deux précurseurs possibles du cholesta 5,22 diéneol sont le cholesta 5,7,22 triénol et le cholesta 5,22, 24 triénol. Ce dernier est absent au début de l'induction (figure 4) alors que le premier est présent puis diminue rapidement en parallèle avec la stabilisation de la formation de cholesta 5,22 dienol. Il peut en être conclu que la source de cholestérol est la réduction du 5,24 diéneol par la A 24-réductase alors que la formation du cholesta 5,22 dienol résulte de la réduction du 5,7,22 triénol par la Δ 7réductase. La formation de cholesta 5,22 dienol par action de la Δ22- desaturase sur le cholestérol n'est pas totalement exclue mais semble un processus minoritaire sur la base de l'accumulation préférentielle de cholestérol par rapport au cholesta 5,22 diéneol au temps long (24h) de la cinétique (figure 4 et 5).

### EXEMPLE 15 : Optimisation de la voie de biosynthèse du cholestérol, rôle de la Δ22-desaturase :

Pour des durées d'induction de l'ordre de 24 h de la souche WGIF04 l'accumulation de cholesta 5,22 diéneol représente environ 50 % de celle du cholestérol au niveau des stérols libres (figure 4). La disruption du gène de la Δ22-desaturase est une option pour optimiser la production de cholestérol. La construction d'une souche doublement disruptée au niveau de la Δ 22-désaturase (gène *erg5*) et du gène *erg6* et exprimant la Δ7-réductase et la Δ24-réductase est parfaitement envisageable. Une souche portant le sous-ensemble : disruption de la Δ 22-desaturase, expression de la Δ7-réductase et expression de la Δ24-réductase a été réalisée.

Cette souche a été obtenue par transformation de la souche CA10 par le plasmide pYES_Delta24 par la technique du chlorure de lithium et par sélection pour la prototrophie envers l'uracile. La souche obtenue a été nommée CA10/Δ24.

La souche CA10 exprimant la Δ24 stérol réductase produit une quantité relativement faible de cholestérol (cf. figure 6 et 7) et accumule de manière majeure de l'ergosta 5-eneol et de manière intermédiaire de l'ergosta 5,7 dieneol. L'accumulation de cholesta 5,7 dienol est très faible dans une telle souche indiquant que la disruption du gène *erg6* est indispensable à l'accumulation importante de dérivés de la série cholesta. L'activité de la Δ 24-réductase est donc, de manière inattendue, peu compétitive avec celle du produit du gène *erg6.* Il peut donc être conclu de ces résultats que la disruption simultanée des gènes *erg5* et *erg6* est importante dans le but d'optimiser la production de cholestérol.

La disruption du gène *erg5* dans la souche WGIF04 permettrait à l'homme du métier d'augmenter de manière importante la production en cholestérol. La faisabilité d'une telle souche est établie par la construction des souches WGIF04 et CA10/Δ24 et le fait que cette souche n'est qu'une combinaison génétique évidente à réaliser des deux souches précédentes. Les donnés issues de WGIF04 indiquent la disparition rapide du cholesta. 5,24 lors de l'expression de la Δ 24-réductase ( en comparant les résultats obtenus avec WGIF03 et ceux obtenus avec WGIF04). Ceci permet de prédire une synthèse très efficace de cholestérol dans une souche disruptée simultanément pour les gènes *erg5* et *erg6,* et co-exprimant la Δ7-réductase et la Δ24-réductase, le cholestérol étant alors le seul stérol terminal.

En conclusion le minimum requis pour la production de cholestérol à un seuil supérieur ou égal à 20% des stérols totaux est la disruption du gène *erg6,* l'expression de la Δ7-réductase et de la Δ24-réductase. La disruption complémentaire de la Δ 22-desaturase permettrait d'améliorer la productivité en cholestérol et d'éliminer la formation parasite de cholesta 5, 22 diéneol comme stérol terminal.

### EXEMPLE 16: Marquage isotopique du cholestérol et définition de signatures isotopiques :

Le principe de l'obtention d'un cholestérol marqué est décrit à la figure 10. Cette manipulation consiste à tout d'abord faire croître la levure sur du glucose totalement marqué au ¹³C pendant 72 heures de culture à 30°C.

Les cellules sont ensuite récupérées par centrifugation du milieu à 600 g pendant 10 minutes. Le culot de cellules est ensuite suspendu dans 50ml de milieu Kappeli frais contenant 2% de galactose non marqué au carbone ¹³C. Les cultures sont arrêtées 2 heures, 4 heures, 8 heures ou 24 heures après le passage en galactose, les stérols sont extraits puis analysés (cf. exemple 7). Le passage de glucose en galactose provoque l'induction des promoteurs GAL10/CYC1 contrôlant à la fois le gène de la Δ7-réductase et celui de la Δ 24-réductase. Simultanément il y a changement de marquage isotopique de la source de carbone. Il s'ensuit la synthèse *de nove* d'intermédiaires métaboliques puis de stérol, incluant le cholestérol et comportant un changement progressif de marquage. Ce changement de marquage peut être caractérisé par le profil de masse de chaque stérol intermédiaire. En effet l'incorporation de chaque atome de ¹³c induit un décalage de masse d'une unité de masse atomique (UMA). Ainsi par exemple le cholestérol apparaît avec une masse molaire variant de 386 à 413 daltons selon le niveau de marquage. En analyse par HPLC-masse, en utilisant l'ionisation electrospray positive, ceci correspond à des m/z (masse/charge) variant de 369 à 396 (ion M+ H⁺ - H₂O, soit M+1-18=385-17=369). Le temps de rétention des stérols en HPLC ne dépendant pas de manière détectable du niveau de marquage, le spectre de masse d'un pic HPLC correspondant à un stérol « X » unique, correspond à une distribution de masse qui est donc la superposition (la somme) des distributions de masse du stérol « X » synthétisé à différents temps après le marquage. Il s'agit donc d'un profil complexe (figure 11), mais parfaitement déterminable expérimentalement, qui représente une signature isotopique unique qui dépend à la fois :
- 1) du protocole de marquage et en particulier des durées et des conditions de culture en ¹²C glucose et en ¹³C galactose.
- 2) de la structure génétique précise de la souche utilisée
- 3) du temps précis d'arrêt des cultures

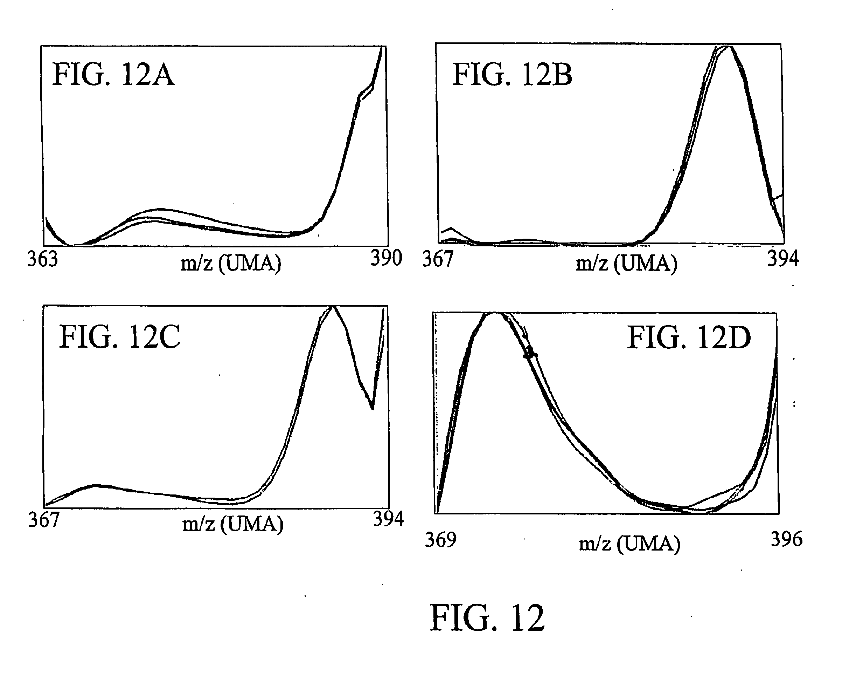

Ce profil isotopique a plusieurs propriétés uniques :
- 1) il est modulable à volonté en jouant sur les conditions de culture, la souche utilisée et le stérol choisi. Un registre de marqueurs unique peut donc être produit.
- 2) il est « combinable » à savoir que plusieurs signatures isotopiques correspondants à plusieurs stérols uniques marqués par des profils isotopiques eux-même modulables peuvent être combinés pour former un « alphabet moléculaire »
- 3) il est reproductible et facile à déterminer expérimentalement (cf. figure 11 et 12) les double ou triple traces qui indiquent la reproductibilité des profils.
- 4) il correspond à un mélange moléculaire traceur facile à isoler, stable, incolore et inodore, non volatile, non toxique et incorporable à des aliments, médicament, additifs ou autres produits assimilables par l'homme.
- 5) il est infalsifiable sauf à disposer des souches recombinantes spécifiques et des conditions très précises de marquage, de culture et d'extraction. De plus la connaissance de la signature isotopique ne permet pas de remonter aux paramètres qui ont permis de la produire.

En résumé un « alphabet isotopique » infalsifiable, d'usage général et incorporable dans tout type de produits, y compris consommables est inventé. Les « mots isotopiques » constituables à partir d'un tel alphabet sont en nombre quasi illimité en mettant à profit à la fois les profils de marquage et les différents types de stérols. L'incorporation de telles signatures au sein de produits les plus variés constitue donc une méthode unique de marquage infalsifiable, contrairement par exemple aux signatures ADN qui peuvent être reproduites une fois connues. La signature peut être lue par ailleurs de manière non destructive, par exemple par ionisation laser suivie d'analyse par spectrométrie de masse (MALDI-TOF ou assimilé).

### EXEMPLE 17 : Production de cholestérol non animal hautement marqué au ¹³C :

L'utilisation de galactose ¹³C ou de glucose et d'éthanol ¹³C en lieu et place des sources de carbone non marqué pour la culture de la souche WGIF04 dans les conditions décrites précédemment pour les analyses de stérols permet la synthèse de stérols et en particulier de cholestérol très fortement marqué (comprenant au moins 95 % de carbone ¹³C). La préparation de stérols et de cholesterol radioactif ¹⁴C est également possible par la même approche. Le procédé peut aussi être incorporé à des souches de levures produisant des stéroïdes et notamment de l'hydrocortisone (cf. demande de brevet WO 02/061109) pour produire des stéroïdes marqués au ¹³C ou au ¹⁴C par exemple pour des tests RIA.

### EXEMPLE 18 : Construction d'une souche produisant majoritairement du cholestérol :

La souche CDR07 Mata est décrite dans la demande de brevet publiée sous le numéro WO02061109 et a été déposée à la Collection Nationale de Cultures de Microorganismes (CNCM), 25 rue du Docteur Roux, 75724 Paris Cedex 15, France, selon les dispositions du Traité de Budapest à la CNCM le 24 janvier 2001 sous le numéros d'ordre I-2616.

La souche CDR07 MATa (marqueurs pertinents : *ade2::GAL10*/*CYC1*ₚ*::*Δ⁷ *; erg5::PGK1*ₚ*::*hygro^{R}; *ERG6*) a été croisée avec la souche WGIF01 décrite dans l'exemple 1 (marqueurs pertinents : *ERG5; erg6::TRP1*)*.*

Après sporulation du diploïde, la composition en stérol des spores est déterminée pour trouver des spores produisant du desmostérol qui est le précurseur du cholestérol comme décrit dans l'exemple 6. On a ainsi identifié une spore produisant du desmostérol et possédant un gène *TRP1* fonctionnel et portant le cDNA, de la Δ7 réductase d*'Arabidopsis thaliana,* ainsi qu'indiqué par l'analyse par PCR. De plus, cette souche, dénommée YIM59, est sensible à l'hygromycine indiquant que le gène *ERG5* est fonctionnel.

Une préparation stérolique, préparée comme décrit dans les exemples 6 et 9, a montré que cette souche YIM59 produit des stérols ayant le même temps de rétention que le zymostérol et le desmostérol. La souche YIM59 a également montré des auxotrophies pour l'adénine, la leucine, l'uracile et l'histidine. La présence de desmostérol démontre que cette souche exprime la stérol Δ7 réductase et qu'elle porte un allèle non fonctionnel *erg6.*

Dans le but d'améliorer le niveau d'expression de la DHCR24 humaine, la séquence nucléotidique du cDNA de la DHCR24 a été modifiée ; afin d'améliorer la traduction au niveau de l'ATG initiateur, le promoteur contrôlant l'expression du DHCR24 a également été modifié. Le nouveau promoteur choisi est le promoteur *CYC1* du cytochrome c1 en remplacement du promoteur inductible *GAL1* du plasmide pYES_Delta24 (cf. exemple 3).

La séquence correspondant au NH2 terminal de la DHCR24 réductase en fusion avec le promoteur CYC1 a été modifiée comme suit :

En petit caractère, on retrouve la séquence nucléotidique partielle du promoteur *CYC1* puis les séquences de recombinaison AttB 1 ensuite une séquence AAAA qui précède l'ATG initiateur. La séquence des deux premiers codons a également été modifiée (séquence GAA-CCT après le codon inititateur ATG).

Le plasmide final portant le cDNA de la DHCR24 sous contrôle du promoteur CYC1 ainsi que l'origine de réplication 2µ de *S.cerevisiae* et le marqueur de sélection URA3-d a été nommé pIM331. Son équivalent sans le cDNA de la DHCR24 a été dénommé pIM303.

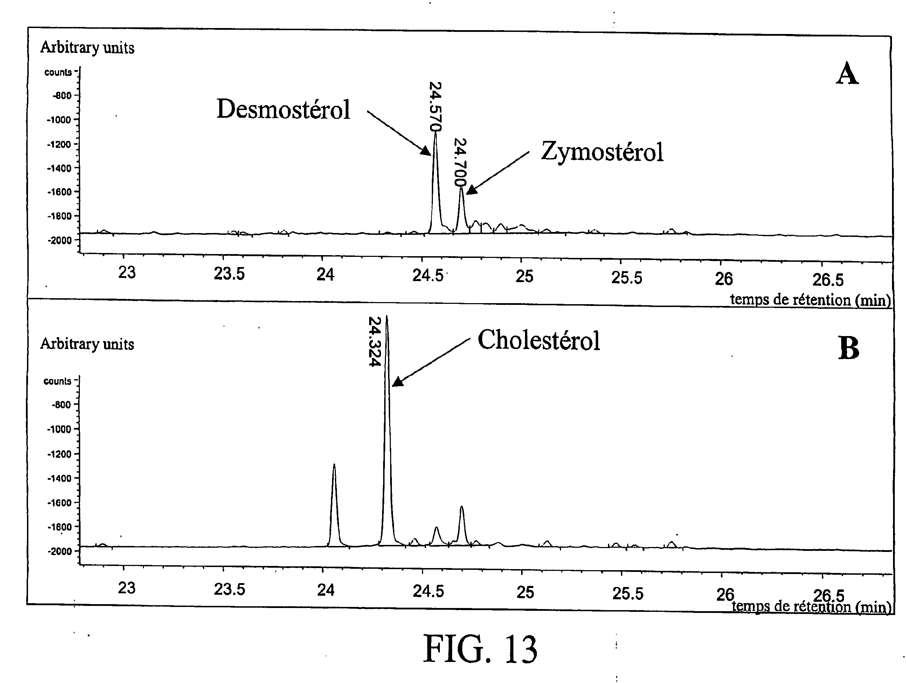

La souche YIM59 est transformée indépendamment par les plasmides pIM303 et pIM331 et deux transformants portant le plasmide pIM303 (souche YIM59/pIM303) ou pIM331 (souche YIM59/pIM331) sont plus particulièrement sélectionnés. Ces souches sont cultivées dans un milieu riche reconstitué de type Kappeli pendant 72 heures à 28°C pour atteindre une absorbance de 40 à 600nm. Des extraits stéroliques totaux (stérols estérifiés et stérols libres) de la souche YIM59/pIM303 (ne portant pas le cDNA de la DHCR24) et de la souche YIM59/pIM331 (portant le cDNA de la DHCR24) sont réalisés en présence de potasse méthanolique (cf. Exemples 5 et 6). Ces deux souches sont testées pour leur capacité à produire du cholestérol. Une partie des résultats (GC) est présentée en figure 13. Les temps de rétention présentés sont donnés en minutes sur les deux chromatogrammes.

Il a ainsi pu être montré que la souche qui ne porte pas le vecteur d'expression de la DHCR24 (souche YIM59/pIM303) ne produit pas de cholestérol (partie A) mais principalement du desmostérol (partie A). A l'inverse, la souche qui porte le cDNA de la DHCR24 (souche YIM59/pIM331) produit un stérol ayant le temps de rétention du cholestérol (partie B). Il a pu être démontré par des techniques de chromatographie en phase gazeuse couplée à un spectromètre de masse par impact électronique (comme décrit à l'exemple 6) que ce stérol est bien du cholestérol. En utilisant les surfaces de chacun des pics de stérol, il a pu être estimé à 57% des stérols la quantité de cholestérol produite par la souche YIM59/pIM331.

### Dépôt de matériel biologique

Les organismes suivants ont été déposés le 22 avril 2004 auprès de la Collection Nationale de Cultures de Microorganismes (CNCM), 25 rue du Docteur Roux, 75724 Paris Cedex 15, France, selon les dispositions du Traité de Budapest.
- Souche WGIF04 déposée sous le numéro d'enregistrement I-3203.

Toutes les publications et brevets cités sont incorporés à la présente demande par référence.

### Bibliographie :

- Adams, A.K., and C. Holm. 1996. Mol Cell Biol. 16:4614-20.
- Ausubel Fred M., Brent Roger, Kingston Robert E., Moore David D., Seidman J.G., Smith John A., Struhl Kevin (Editeurs). Current Protocols in Molecular Biology, publié par John Wiley & Sons Inc. Current Protocols Customer Service, 605 Third Avenue, 9th Floor New York, NY 10158 USA (édition mise à jour de Mars 2002).
- Baudin-Baillieu, A., E. Guillemet, C. Cullin, and F. Lacroute. 1997. Yeast. 13:353-6.
- Bonneaud, N., O. Ozier-Kalogeropoulos, G.Y. Li, M. Labouesse, L. Minvielle-Sebastia, and F. Lacroute. 1991. Yeast. 7:609-15.
- Bowman, S. et al.. 1997. Nature. 387:90-3.
- Duport, C., B. Schoepp, E. Chatelain, R. Spagnoli, B. Dumas, and D. Pompon. 2003. Eur J Biochem. 270:1502-14.
- Gietz, R.D., R.H. Schiest1, A.R. Willems, and R.A. Woods. 1995. Yeast. 11:355-60.
- Goffeau, A., et al.. 1996. Science. 274:546, 563-7.
- Jensen-Pergakes, K.L., M.A. Kennedy, N.D. Lees, R. Barbuch, C. Koegel, and M. Bard. 1998. Antimicrob Agents Chemother. 42:1160-7.
- Kaneshiro, E.S., J.A. Rosenfeld, M. Basselin-Eiweida, J.R. Stringer, S.P. Keely, A.G. Smulian, and J.L. Giner. 2002. Mol Microbiol. 44:989-99.
- Kappeli, O., M. Arreguin, and M. Rieger. 1985. J Gen Microbiol. 131:1411-6*.*
- Kelly, S.L., D.C. Lamb, B.C. Baldwin, A.J. Corran, and D.E. Kelly. 1997. J Biol Chem. 272:9986-8.
- Lecain, E., X. Chenivesse, R. Spagnoli, and D. Pompon. 1996. J Biol Chem. 271:10866-73.
- McCammon, M.T., M.A. Hartmann, C.D. Bottema, and L.W. Parks. 1984. J Bacteriol. 157:475-83.
- Moebius, F.F., B.U. Fitzky, J.N. Lee, Y.K. Paik, and H. Glossmann. 1998. Proc Natl Acad Sci U S A. 95:1899-902.
- Noda, H., and Y. Koizumi. 2003. Insect Biochem Mol Biol. 33:649-58.
- Ozier-Kalogeropoulos, O., A. Malpertuy, J. Boyer, F. Tekaia, and B. Dujon. 1998. Nucleic Acids Res. 26:5511-24.
- Skaggs, B.A., J.F. Alexander, C.A. Pierson, K.S. Schweitzer, K.T. Chun, C. Koegel, R. Barbuch, and M. Bard. 1996. Gene. 169:105-9.
- Sturley, S.L. 2000. Biochim Biophys Acta. 1529:155-63.
- Szczebara, F.M., et al.. 2003. Nat Biotechnol. 21:143-9.
- Tait, E., M.C. Simon, S. King, A.J. Brown, N.A. Gow, and D.J. Shaw. 1997. Fungal Genet Biol. 21:308-14.
- Taton, M., and A. Rahier. 1991. Biochem Biophys Res Commun. 181:465-73.
- Taylor, F.R., and L.W. Parks. 1978. J Bacteriol. 136:531-7.
- Waterham, H.R., J. Koster, G.J. Romeijn, R.C. Hennekam, P. Vreken, H.C. Andersson, D.R. FitzPatrick, R.I. Kelley, and R.J. Wanders. 2001. Am J Hum Genet. 69:685-94.
- Wood, V., et al. 2002. Nature. 415:871-80.

**Table 1 : Evaluation des contributions spectrales des insaturations des stérols (coefficients d'extinction mM-1cm-1)**

| **Insaturation** | Longueur d'onde (nm) | | |
|---|---|---|---|
| | 206 | 235 | 280 |
| **5** | 3,9 | 0,0 | 0,0 |
| **5,7** | 4,0 | 1,7 | 11,5 |
| **5,7,22** | 4,8 | 1,7 | 11,5 |
| **5,22** | 4,4 | 0,0 | 0,0 |
| **5,24** | 6,9 | 0,0 | 0,0 |
| **5,22,24** | 8,7 | 27,0 | 0,0 |
| **5,7,24** | 7,5 | 1,7 | 11,5 |
| **5,7,22,24** | 9,2 | 29,8 | 11,5 |
| **8,24** | 6,9 | 0,0 | 0,0 |

## Revendications

1. Souche de levure produisant de manière autonome du cholestérol.
à partir d'une source de carbone simple,
**caracterisée en ce qu'**elle exprime les enzymes hétérologues 7-déhydrocholestérol réductase et 3β-hydroxystérol Δ24-réductase, et **en ce que**
l'enzyme stérol 24-C-méthyltransferase a été inactivée.

2. Souche de levure selon la revendication 1 dans laquelle l'enzyme C-22 stérol désaturase a été inactivée.

3. Souche de levure selon l'une des revendications 1 ou 2 **caractérisée en ce que** l'expression des enzymes 7-déhydrocholestérol réductase et 3β-hydroxystérol Δ24-réductase est obtenue par transformation de l'organisme.

4. Souche de levure selon l'une des revendications 1 à 3 **caractérisée en ce que** l'inactivation de l'enzyme stérol 24-C-méthyltransferase est réalisée par inactivation génique.

5. Souche de levure selon la revendication 2 **caractérisée en ce que** l'inactivation de l'enzyme C-22 stérol désaturase est réalisée par inactivation génique.

6. Souche de levure selon l'une des revendications précédentes **caractérisée en ce qu'**elle est choisie dans le genre *Saccharomyces* ou *Schizosaccharomyces.*

7. Souche de levure *Saccharomyces cerevisiae* **caractérisée en ce qu'**il s'agit de la souche WGIF04 déposée auprès de la Collection Nationale de Cultures de Microorganismes (CNCM) le 22 avril 2004 sous le numéro d'enregistrement I-3203.

8. Procédé de production de cholestérol d'origine non animal comprenant la culture d'un organisme selon l'une des revendications précédentes.

9. Procédé selon la revendication 8 **caractérisé en ce que** l'étape de culture de l'organisme est suivie d'une étape d'extraction du cholestérol.

10. Procédé selon la revendication 9 **caractérisé en ce que** l'extraction du cholestérol est effectuée par un solvant non miscible dans l'eau.

11. Procédé selon la revendication 9 ou 10 **caractérisé en ce que** l'on effectue une étape de saponification avant l'extraction du cholestérol.

12. Procédé selon la revendication 9, 10 ou 11 **caractérisé en ce que** l'on effectue une étape de broyage mécanique des cellules avant la saponification ou l'extraction du cholestérol.

13. Utilisation d'un organisme selon l'une des revendications 1 à 7 pour la production de cholestérol, ou de l'un de ses d'intermédiaires métaboliques, ou d'un mélange de stérols marqué au ¹³C ou au ¹⁴C.

14. Procédé de production de cholestérol, ou de l'un de ses d'intermédiaires métaboliques, ou d'un mélange de stérols marqué au ¹³C ou au ¹⁴C comprenant les étapes suivantes :
- culture d'un organisme selon l'une des revendications 1 à 7 sur un substrat marqué au ¹³C ou au ¹⁴C et,
- extraction dudit cholestérol, ou de l'un de ses intermédiaires métaboliques, ou du mélange de stérols.

15. Procédé de fabrication d'un mélange isotopique de cholestérol, d'intermédiaires ou de métabolites du cholestérol, marqués en différentes positions à l'aide de marqueurs isotopiques, comprenant la mise en culture d'un organisme selon l'une des revendications 1 à 7 sur un substrat marqué puis sur un substrat non marqué, les durées de culture sur chacun de ces substrats étant choisies afin d'obtenir un profil isotopique défini.

## Claims

1. Yeast strain which autonomously produces cholesterol from a simple carbon source, **characterized in that** it expresses the heterologous enzymes 7-dehydrocholesterol reductase and 3β-hydroxysterol Δ24-reductase, and **in that** the enzyme sterol 24-C-methyltransferase has been inactivated.

2. Yeast strain according to Claim 1, in which the enzyme C-22 sterol desaturase has been inactivated.

3. Yeast strain according to either of Claims 1 and 2, **characterized in that** the expression of the enzymes 7-dehydrocholesterol reductase and 3β-hydroxysterol Δ24-reductase is obtained by transforming the organism.

4. Yeast strain according to one of Claims 1 to 3, **characterized in that** the inactivation of the enzyme sterol 24-C-methyltransferase is carried out by gene inactivation.

5. Yeast strain according to Claim 2, **characterized in that** the inactivation of the C-22 sterol desaturase enzyme is carried out by gene inactivation.

6. Yeast strain according to one of the preceding claims, **characterized in that** it is chosen from the genus *Saccharomyces* or *Schizosaccharomyces.*

7. *Saccharomyces cerevisiae* yeast strain, **characterized in that** it is the strain WGIF04 deposited with the Collection Nationale de Cultures de Microorganismes (CNCM) [National Collection of Cultures and Microorganisms] on April 22, 2004, under the registration number I-3203.

8. Method for producing cholesterol of nonanimal origin, comprising the culturing of an organism according to one of the preceding claims.

9. Method according to Claim 8, **characterized in that** the step for culturing the organism is followed by a step for extracting the cholesterol.

10. Method according to Claim 9, **characterized in that** the extraction of the cholesterol is carried out with a water-immiscible solvent.

11. Method according to Claim 9 or 10, **characterized in that** a saponification step is carried out before the extraction of the cholesterol.

12. Method according to Claim 9, 10 or 11, **characterized in that** a step comprising mechanical grinding of the cells is carried out before the saponification or the extraction of the cholesterol.

13. Use of an organism according to one of Claims 1 to 7, for producing cholesterol, or one of its metabolic intermediates, or a mixture of sterols labeled with ¹³C or with ¹⁴C.

14. Method for producing cholesterol, or one of its metabolic intermediates, or a mixture of sterols labeled with ¹³C or with ¹⁴C, comprising the following steps:
- culturing an organism according to one of Claims 1 to 7 on a ¹³C- or ¹⁴C-labeled substrate, and
- extracting said cholesterol, or one of its metabolic intermediates, or the mixture of sterols.

15. Method for producing an isotopic mixture of cholesterol, or of cholesterol intermediates or metabolites, labeled at various positions using isotope labels, comprising the placing of an organism according to one of Claims 1 to 7 in culture on a labeled substrate and then on a nonlabeled substrate, the culturing times on each of these substrates being chosen in order to obtain a defined isotope profile.

## Patentansprüche

1. Hefestamm, der ausgehend von einer einfachen Kohlenstoffquelle autonom Cholesterol produziert, **dadurch gekennzeichnet, dass** er die heterologen Enzyme 7-Dehydrocholesterolreduktase und 3β-Hydroxysterol-Δ24-Reduktase exprimiert und dass das Enzym Sterol-24-C-Methyltransferase inaktiviert wurde.

2. Hefestamm nach Anspruch 1, in dem das Enzym C-22-Steroldesaturase inaktiviert worden ist.

3. Hefestamm nach einem der Ansprüche 1 oder 2, **dadurch gekennzeichnet, dass** die Expression der Enzyme 7-Dehydrocholesterolreduktase und 3β-Hydroxysterol-Δ24-Reduktase durch Transformation des Organismus erzielt wird.

4. Hefestamm nach einem der Ansprüche 1 bis 3, **dadurch gekennzeichnet, dass** die Inaktivierung des Enzyms Sterol-24-C-Methyltransferase durch Geninaktivierung erfolgt.

5. Hefestamm nach Anspruch 2, **dadurch gekennzeichnet, dass** die Inaktivierung des Enzyms C-22-Steroldesaturase durch Geninaktivierung erfolgt.

6. Hefestamm nach einem der vorhergehenden Ansprüche, **dadurch gekennzeichnet, dass** er aus der Gattung *Saccharomyces* oder *Schizosaccharomyces* stammt.

7. *Saccharomyces-cerevisiae*-Hefestamm, **dadurch gekennzeichnet, dass** es sich um den am 22. April 2004 unter der Hinterlegungsnummer I-3203 bei der "Collection Nationale de Cultures de Microorganismes" (CNCM) hinterlegten Stamm WGIF04 handelt.

8. Verfahren für die Herstellung von Cholesterol nicht tierischen Ursprungs, bei dem man einen Organismus nach einem der vorhergehenden Ansprüche kultiviert.

9. Verfahren nach Anspruch 8, **dadurch gekennzeichnet, dass** sich an den Schritt, in dem der Organismus kultiviert wird, ein Schritt, in dem das Cholesterol extrahiert wird, anschließt.

10. Verfahren nach Anspruch 9, **dadurch gekennzeichnet, dass** die Extraktion des Cholesterols mit einem nicht mit Wasser mischbaren Lösungsmittel erfolgt.

11. Verfahren nach Anspruch 9 oder 10, **dadurch gekennzeichnet, dass** man vor der Extraktion des Cholesterols einen Verseifungsschritt durchführt.

12. Verfahren nach Anspruch 9, 10 oder 11, **dadurch gekennzeichnet, dass** man vor der Verseifung oder Extraktion des Cholesterols einen Schritt, in dem die Zellen mechanisch aufgebrochen werden, durchführt.

13. Verwendung eines Organismus nach einem der Ansprüche 1 bis 7 für die Produktion von Cholesterol oder von einem seiner Stoffwechselzwischenprodukte oder von einer Mischung von Sterolen, markiert mit ¹³C oder mit ¹⁴C.

14. Verfahren für die Produktion von Cholesterol oder von einem seiner Stoffwechselzwischenprodukte oder von einer Mischung von Sterolen, markiert mit ¹³C oder mit ¹⁴C, umfassend die folgenden Schritte:
- Kultivieren eines Organismus nach einem der Ansprüche 1 bis 7 auf einem mit ¹³C oder ¹⁴C markierten Substrat,
- Extrahieren des Cholesterols oder des einen seiner Stoffwechselzwischenprodukte oder der Sterolmischung.

15. Verfahren zur Herstellung einer isotopen Mischung von Cholesterol, Zwischenprodukten oder Metaboliten des Cholesterols, die in verschiedenen Positionen mit Isotopenmarkern markiert sind, umfassend das Kultivieren eines Organismus nach einem der Ansprüche 1 bis 7 auf einem markierten Substrat und dann auf einem nicht markierten Substrat, wobei die Kulturdauer auf jedem der Substrate so gewählt ist, dass man zu einem definierten Isotopenprofil gelangt.
